(19) Europäisches Patentamt
European Patent Office
Office européen des brevets

(11) Veröffentlichungsnummer: **0 045 452**
**A2**

(12) **EUROPÄISCHE PATENTANMELDUNG**

(21) Anmeldenummer: **81105835.3**

(22) Anmeldetag: **23.07.81**

(51) Int. Cl.³: **C 07 D 243/24**

(30) Priorität: **31.07.80 CH 5841/80**

(43) Veröffentlichungstag der Anmeldung:
**10.02.82 Patentblatt 82/6**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Branca, Quirico, Dr.**
**Lenzgasse 2**
**CH-4056 Basel(CH)**

(72) Erfinder: **Fischli, Albert Eduard, Prof. Dr.**
**Am Ausserberg 20**
**CH-4125 Riehen(CH)**

(72) Erfinder: **Szente, André, Dr.**
**Baselstrasse 70**
**CH-4125 Riehen(CH)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80(DE)**

(54) **Benzodiazepin-Derivate, Zwischenprodukte und Verfahren zu deren Herstellung, sowie diese enthaltende Arzneimittel.**

(57) Die neuen Benzodiazepin-Derivate der allgemeinen Formel

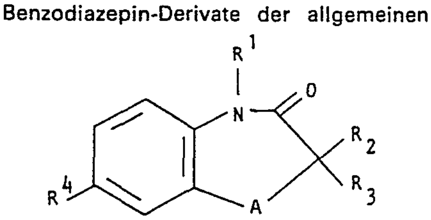

worin A die Gruppe

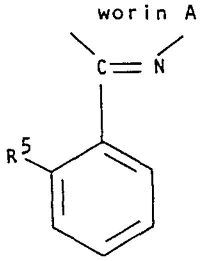

(a),

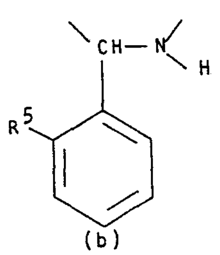

(b) oder

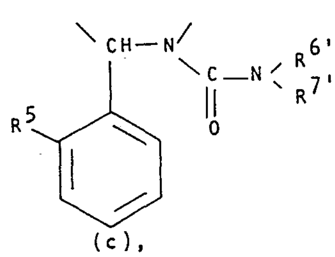

(c),

bedeutet und R¹, R², R³, R⁴, R⁵, R⁶, und R⁷ die in Anspruch 1 angegebene Bedeutung besitzen, haben ausgeprägte aldosteronantagonistische Eigenschaften und eignen sich zur Bekämpfung bzw Verhutung von Herzversagen, hepatitischer Aszites, primärem Aldosteronismus und essentielle Hypertonie.

Sie können ausgehend von teilweise neuen Zwischenprodukten nach verschiedenen Verfahren hergestellt werden. Ihre Verwendung als Heilmittel wird beschrieben.

EP 0 045 452 A2

COMPLETE DOCUMENT

0045452

23. Juli 1981

F.Hoffmann-La Roche & Co.Aktiengesellschaft, Basel/Schweiz

RAN 4008/314

# Benzodiazepin-Derivate, Zwischenprodukte und Verfahren zu deren Herstellung sowie diese enthaltende Arzneimittel

Die vorliegende Erfindung betrifft neue Benzodiazepin-Derivate der allgemeinen Formel

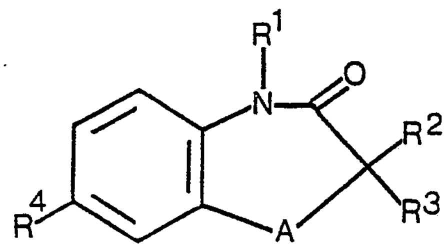

I

worin A die Gruppe

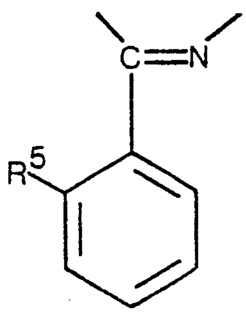

(a),

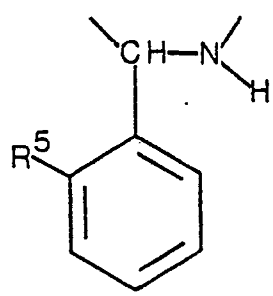

(b) oder

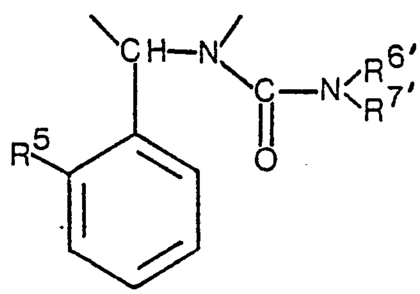

(c),

$R^1$ niederes Alkyl, $R^2$ und $R^3$ je Wasserstoff oder niederes Alkyl, $R^4$ die Gruppe

Nt/30.4.1981

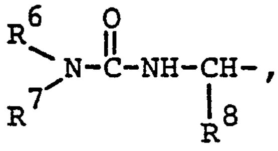 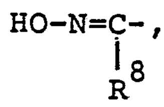 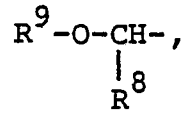 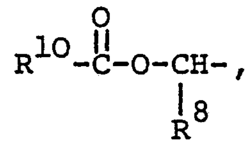

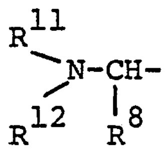 oder 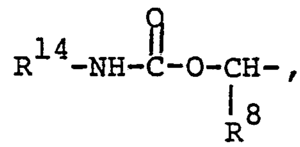

$R^5$ Wasserstoff oder Halogen, $R^8$ Wasserstoff oder niederes Alkyl, $R^9$ niederes Alkyl oder niederes Alkoxyalkyl, $R^{10}$ niederes Alkyl, $R^{11}$ Wasserstoff, niederes Alkyl oder niederes Hydroxyalkyl, $R^{12}$ Wasserstoff oder niederes Alkyl und $R^{14}$ niederes Alkyl oder Aryl bedeuten, und entweder $R^6$ Wasserstoff oder niederes Alkyl und $R^7$ niederes Alkyl oder niederes Hydroxyalkyl bedeuten oder $R^6$ und $R^7$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13}$ als Ringglied enthalten kann, wobei $R^{13}$ Wasserstoff oder niederes Alkyl bedeutet, und entweder $R^{6'}$ Wasserstoff oder niederes Alkyl und $R^{7'}$ niederes Alkyl bedeuten oder $R^{6'}$ und $R^{7'}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13'}$ als Ringglied enthalten kann, wobei $R^{13'}$ niederes Alkyl bedeutet, mit der Massgabe, dass $R^4$ die Gruppe $R^{6'}R^{7'}N-CO-NH-CH(R^8)-$ bedeutet, wenn A die Gruppe (c) bedeutet,

und pharmazeutisch annehmbare Säureadditionssalze davon.

Gegenstand der vorliegenden Erfindung sind Benzodiazepin-Derivate der obigen allgemeinen Formel I und ihre pharmazeutisch annehmbaren Säureadditionssalze als solche und als pharmazeutische Wirkstoffe, die Herstellung dieser Verbindungen und Zwischenprodukte für die Herstellung

dieser Verbindungen, ferner Arzneimittel, enthaltend eine oder mehrere Verbindungen der allgemeinen Formel I oder pharmazeutisch annehmbare Säureadditionssalze davon und die Herstellung solcher Arzneimittel, sowie die Verwendung von Benzodiazepin-Derivaten der allgemeinen Formel I und von pharmazeutisch annehmbaren Säureadditionssalzen davon bei der Bekämpfung bzw. Verhütung von Krankheiten.

Der Ausdruck "niederes Alkyl", für sich allein genommen oder in Kombinationen, wie "niederes Hydroxyalkyl", "niederes Alkoxyalkyl" und dergleichen, bezeichnet geradkettige oder verzweigte gesättigte Kohlenwasserstoffreste mit höchstens 7, vorzugsweise höchstens 4 Kohlenstoffatomen, wie Methyl, Aethyl, n-Propyl, Isopropyl, n-Butyl, t-Butyl usw. Der Ausdruck "niederes Hydroxyalkyl" umfasst Reste wie 2-Hydroxyäthyl, 3-Hydroxy-2-propyl und dergleichen. Der Ausdruck "niederes Alkoxyalkyl" umfasst Reste wie Methoxymethyl, Aethoxymethyl, Methoxy-2-propyl und dergleichen. Der Ausdruck "Halogen" bedeutet Fluor, Chlor, Brom oder Jod. Der Ausdruck "Aryl" bedeutet Phenyl, ggf. substituiert durch niederes Alkyl, Halogen, Nitro oder niederes Alkoxy. Wenn $R^6$ und $R^7$ zusammen mit dem Stickstoffatom einen 3- bis 7-gliedrigen Heterocyclus bedeuten, so kommen in erster Linie Aziridin-, Pyrrolidin-, Piperidin-, Morpholin- und Piperazinreste in Betracht.

In einem speziellen Aspekt umfasst die vorliegende Erfindung Verbindungen der obigen allgemeinen Formel I, worin entweder $R^1$ und $R^2$ beide Methyl bedeuten oder $R^1$ Aethyl und $R^2$ Wasserstoff bedeuten, wenn $R^3$ Wasserstoff, $R^4$ die Gruppe $HO-N=C(CH_3)-$, A die Gruppe (a) und $R^5$ Fluor bedeuten. In einem weiteren speziellen Aspekt umfasst die vorliegende Erfindung Verbindungen der obigen allgemeinen Formel I, worin $R^2$ und $R^3$ beide niederes Alkyl bedeuten, wenn A die Gruppe (a), $R^4$ die Gruppe $HO-N=C(R^8)-$ und $R^5$ und $R^8$ obige Bedeutung besitzen.

Unter den Verbindungen, die von der allgemeinen Formel I umfasst werden, sind diejenigen bevorzugt, worin A die Gruppe (a) oder (b) bedeutet. $R^1$ bedeutet vorzugsweise Methyl oder Aethyl. $R^2$ und $R^3$ bedeuten vorzugsweise Wasserstoff oder Methyl. $R^4$ bedeutet vorzugsweise die Gruppe $R^6R^7N-CO-NH-CH(R^8)-$, $HO-N=C(R^8)-$, $R^9-O-CH(R^8)-$ oder $R^{11}R^{12}N-CH(R^8)-$. $R^5$ bedeutet vorzugsweise Wasserstoff oder Fluor. $R^6$ und $R^7$ bedeuten vorzugsweise beide Methyl, oder $R^6$ Wasserstoff und $R^7$ n-Butyl oder 2-Hydroxyäthyl, oder $R^6$ und $R^7$ zusammen mit dem Stickstoffatom Pyrrolidinyl. $R^8$ bedeutet vorzugsweise Wasserstoff oder Methyl. $R^9$ bedeutet vorzugsweise Methoxymethyl. Die bevorzugte Bedeutungsmöglichkeit von $R^{10}$ ist Methyl. $R^{11}$ und $R^{12}$ bedeuten vorzugsweise beide Wasserstoff oder beide Methyl, oder $R^{11}$ Hydroxyäthyl und $R^{12}$ Wasserstoff.

Im Rahmen der vorliegenden Erfindung besonders bevorzugte, von der allgemeinen Formel I umfasste Verbindungen sind:

1-Aethyl-5-(o-fluorphenyl)-1,3-dihydro-7-[1-(methoxymethoxy)äthyl]-2H-1,4-benzodiazepin-2-on;

1-Aethyl-5-(o-fluorphenyl)-1,3,4,5-tetrahydro-7-[1-(hydroxyimino)äthyl]-2H-1,4-benzodiazepin-2-on;

1-Aethyl-5-(o-fluorphenyl)-1,3,4,5-tetrahydro-7-/1-[(2-hydroxyäthyl)amino]äthyl/-2H-1,4-benzodiazepin-2-on;

7-[1-(Dimethylamino)äthyl]-1-äthyl-5-(o-fluorphenyl)-1,3,4,5-tetrahydro-2H-1,4-benzodiazepin-2-on;

7-(1-Aminoäthyl)-5-(o-fluorphenyl)-1,3-dihydro-1,3-dimethyl-2H-1,4-benzodiazepin-2-on und

1-[(2,3-Dihydro-1-methyl-2-oxo-5-phenyl-1H-1,4-benzodiazepin-7-yl)methyl]-3-(2-hydroxäthyl)harnstoff.

Die neuen Benzodiazepin-Derivate der allgemeinen Formel I und ihre pharmazeutisch annehmbaren Säureadditionssalze können erfindungsgemäss hergestellt werden, indem man

a) ein Nitril der allgemeinen Formel

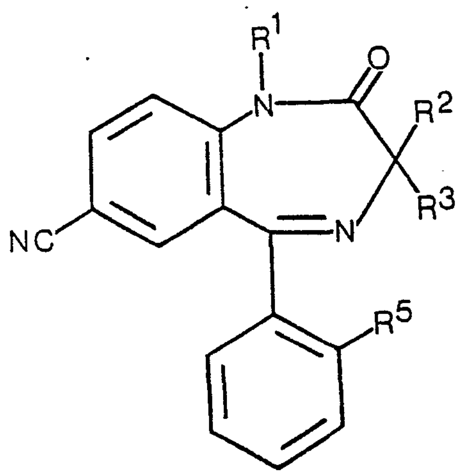

II

worin $R^1$, $R^2$, $R^3$ und $R^5$ obige Bedeutung besitzen, zum entsprechenden primären Amin reduziert, oder

b) eine Carbonylverbindung der allgemeinen Formel

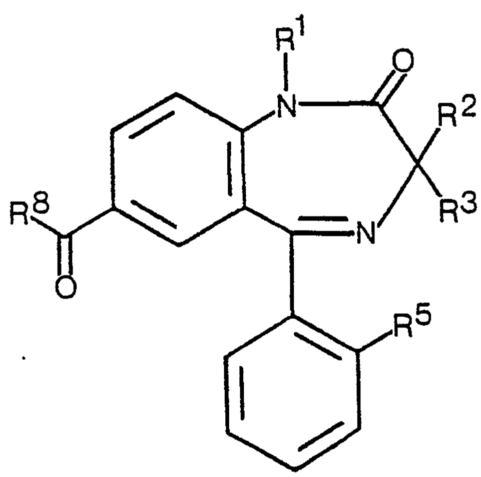

III

worin $R^1$, $R^2$, $R^3$, $R^5$ und $R^8$ obige Bedeutung besitzen, mit Hydroxylamin behandelt, oder

c) eine Carbonylverbindung der obigen allgemeinen Formel III mit einem Amin der allgemeinen Formel

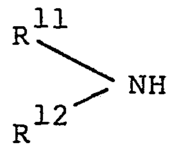

IV

worin $R^{11}$ und $R^{12}$ obige Bedeutung besitzen, und einem Reduktionsmittel umsetzt, oder

d) in einer Verbindung der allgemeinen Formel

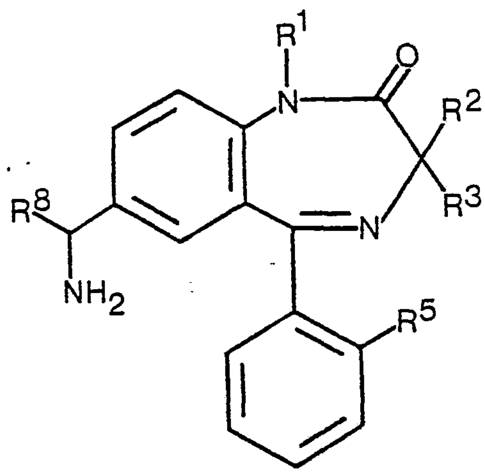

Ib'

worin $R^1$, $R^2$, $R^3$, $R^5$ und $R^8$ obige Bedeutung besitzen, die primäre Aminogruppe mono- oder dialkyliert, oder

e) ein Oxim der allgemeinen Formel

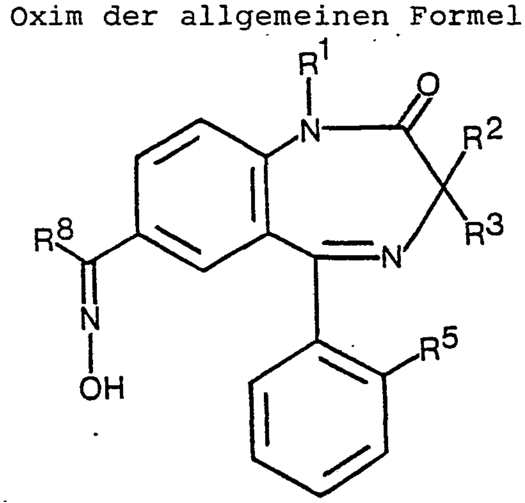

Ia

worin $R^1$, $R^2$, $R^3$, $R^5$ und $R^8$ obige Bedeutung besitzen, zum entsprechenden primären Amin reduziert, oder

f) ein Isocyanat der allgemeinen Formel

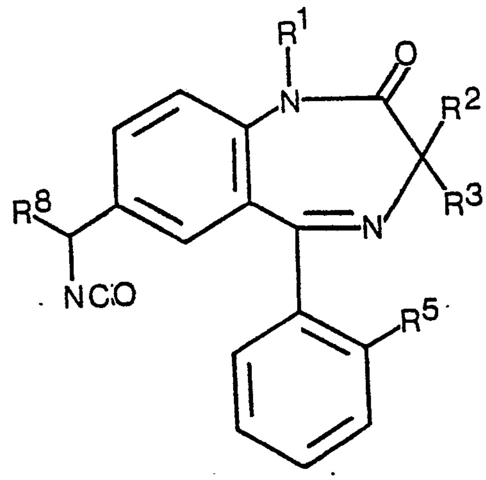

V

worin $R^1$, $R^2$, $R^3$, $R^5$ und $R^8$ obige Bedeutung besitzen, mit einem Amin der allgemeinen Formel

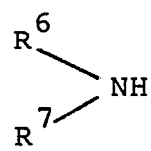

VI

worin $R^6$ und $R^7$ obige Bedeutung besitzen, umsetzt, oder

g) ein Amin der allgemeinen Formel

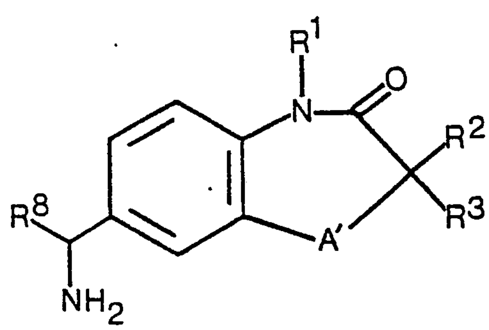

Ib

worin A' die Gruppe (a) oder (b) bedeutet, und $R^1$, $R^2$, $R^3$ und $R^8$ obige Bedeutung besitzen,
mit einem Halogenid der allgemeinen Formel

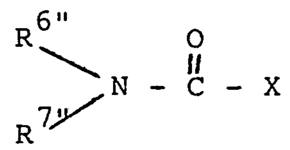

VII

worin X Halogen bedeutet, und entweder $R^{6''}$ und $R^{7''}$ je niederes Alkyl bedeuten oder $R^{6''}$ und $R^{7''}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel >N-$R^{13'}$ als Ringglied enthalten kann, wobei $R^{13'}$ niederes Alkyl bedeutet,
umsetzt, oder

h) ein Amin der obigen allgemeinen Formel Ib mit einem Isocyanat der allgemeinen Formel

$R^{7'''}$ - NCO VIII

worin $R^{7'''}$ niederes Alkyl bedeutet,
umsetzt, oder

i) einen Alkohol der allgemeinen Formel

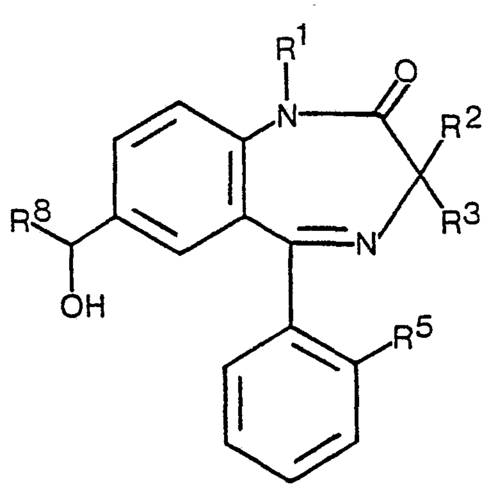

IX

worin $R^1$, $R^2$, $R^3$, $R^5$ und $R^8$ obige Bedeutung besitzen, mit einer Verbindung der allgemeinen Formel

$$R^9 - L \qquad X$$

worin $R^9$ obige Bedeutung besitzt, und L eine Abgangsgruppe bedeutet,

veräthert, oder

j) einen Alkohol der obigen Formel IX mit einem einen Rest der allgemeinen Formel

$$R^{10} - \overset{O}{\overset{\|}{C}} - \qquad XI$$

worin $R^{10}$ obige Bedeutung besitzt,

liefernden Mittel umsetzt, oder

k) einen Alkohol der obigen allgemeinen Formel IX mit einem Isocyanat der allgemeinen Formel

$$R^{14} - NCO \qquad XII$$

worin $R^{14}$ obige Bedeutung besitzt,

umsetzt, oder

l) eine Iminoverbindung der allgemeinen Formel

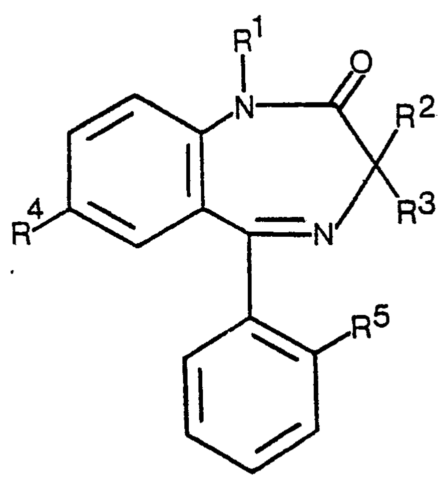

Ic

worin $R^1$, $R^2$, $R^3$, $R^4$ und $R^5$ obige Bedeutung besitzen,

zum entsprechenden Amin reduziert, oder

m) eine Verbindung der allgemeinen Formel I in ein pharmazeutisch annehmbares Säureadditionssalz überführt.

Gemäss Verfahrensvariante a) können Benzodiazepin-Derivate der allgemeinen Formel I hergestellt werden, indem man ein Nitril der allgemeinen Formel II zum entsprechenden primären Amin reduziert. Dabei gilt es zu beachten, dass, je nach Reaktivität des eingesetzten Reduktionsmittels, neben der Nitrilgruppe im gleichen Arbeitsgang auch die im Molekül vorhandene Iminogruppe zur entsprechenden Aminogruppe reduziert werden kann. Wird die Reduktion beispielsweise mit elementarem Wasserstoff in Gegenwart eines nicht besonders reaktiven Katalysators, wie z.B. Raney-Nickel, durchgeführt, und zwar in einem Ammoniak enthaltenden Alkohol, wie z.B. Methanol oder Aethanol und bei Temperaturen von etwa Raumtemperatur bis etwa 70°C, vorzugsweise ca. 50°C, und Drucken von etwa Normaldruck bis etwa 6 atm., vorzugsweise bei 3,5 atm, so kann, je nach Reaktionsdauer die Nitrilgruppe selektiv zum primären Amin reduziert werden. Wird die Reaktion nach Aufnahme von einem Aequivalent Wasserstoff fortgesetzt, so wird auch die im Molekül vorhandene Iminogruppe zum Amin reduziert.

Gemäss Verfahrensvariante b) können Benzodiazepin-Derivate der allgemeinen Formel I hergestellt werden, indem man eine Verbindung der allgemeinen Formel III mit Hydroxylamin behandelt. Diese Reaktion kann nach verschiedenen, allgemein üblichen und jedem Fachmann geläufigen Methoden durchgeführt werden. In einer bevorzugten Ausführungsform setzt man Verbindungen der allgemeinen Formel III in Pyridin oder in einem Gemisch von Pyridin und einem Alkohol, wie z.B. Methanol oder Aethanol, mit Hydroxylamin-hydrochlorid um, und zwar zweckmässigerweise bei Raumtemperatur.

Gemäss Verfahrensvariante c) können Benzodiazepin-Derivate der allgemeinen Formel I hergestellt werden, indem man eine Verbindung der allgemeinen Formel III mit einem Amin der Formel IV und einem Reduktionsmittel umsetzt. Beispielsweise lässt man Verbindungen der allgemeinen Formel III in einem Alkohol, wie z.B.Methanol oder Aethanol, der eine Säure, wie z.B. Salzsäure, enthält, mit einem Amin der Formel IV und einem Reduktionsmittel, wie Natriumcyanoborhydrid, reagieren. In einem ersten Schritt bildet sich dabei aus der Carbonylverbindung der allgemeinen Formel III und dem Amin der Formel IV das entsprechende Imin, das dann im zweiten Schritt zum Amin reduziert wird. Dabei gilt es zu beachten, dass die ursprünglich im Molekül vorhandene Iminogruppe ebenfalls zum entsprechenden Amin reduziert wird.

Gemäss Verfahrensvariante d) können Benzodiazepin-Derivate der allgemeinen Formel I hergestellt werden, indem man in einem Amin der allgemeinen Formel Ib' die primäre Aminogruppe mono- oder dialkyliert. Diese Reaktion kann mit jedem geeigneten Alkylierungsmittel durchgeführt werden, beispielsweise mit einem entsprechenden Halogenid, wie Methyljodid, 2-Bromäthanol, Jodäthan und dergleichen, Dialkylsulfaten, wie z.B. Dimethylsulfat oder Diäthylsulfat, in Gegenwart eines säurebindenden Mittels, mit einem Aldehyd, wie z.B. Formaldehyd und Acetaldehyd, unter reduzierenden Bedingungen, oder mit einer entsprechenden Epoxyverbindung, wie Aethylenoxid und dergleichen.

Die Reaktionsbedingungen können von jedem Fachmann je nach dem zum Einsatz gelangenden Alkylierungsmittel leicht ausgewählt werden. Beispielsweise wird ein Amin der allgemeinen Formel Ib' mit einer äquivalenten Menge Aldehyd in Ameisensäure unter Rückfluss zum Sieden erhitzt bis kein Kohlendioxid mehr entweicht, worauf das Lösungsmittel im Vakuum entfernt und die freie Base durch Neutralisieren isoliert wird.

Gemäss Verfahrensvariante e) können Benzodiazepin-Derivate der allgemeinen Formel I hergestellt werden, indem man ein Oxim der allgemeinen Formel Ia zum entsprechenden primären Amin reduziert. Diese Reaktion erfolgt nach allgemein üblichen, jedem Fachmann geläufigen Methoden, wobei man beachten muss, dass, je nach eingesetztem Reduktionsmittel, auch die im Molekül vorhandene Iminogruppe reduziert werden kann.

Führt man die Reaktion beispielsweise in einem Alkohol, wie z.B. Methanol oder Aethanol, der Ammoniak enthält, mit elementarem Wasserstoff als Reduktionsmittel und Raney-Nickel als Katalysator durch, so kann selektiv die Oximgruppe zum Amin reduziert werden, wenn man bei Raumtemperatur und Normaldruck arbeitet und die Reaktion nach Aufnahme von einem Aequivalent Wasserstoff abbricht.

Gemäss Verfahrensvariante f) können Benzodiazepin-Derivate der allgemeinen Formel I hergestellt werden, indem man ein Isocyanat der allgemeinen Formel V mit einem Amin der Formel VI umsetzt. Zweckmässigerweise geht man dabei so vor, dass man das zum Einsatz vorgesehene Benzodiazepin-Derivat der allgemeinen Formel V kurz oder unmittelbar vor der Reaktion mit der Aminoverbindung der allgemeinen Formel VI auf die weiter unten beschriebene Weise aus einem entsprechenden Benzodiazepin-Derivat der allgemeinen Formel Ib' herstellt und das betreffende Benzodiazepin-Derivat der allgemeinen Formel V nicht in isolierter Form in die Reaktion einbringt, sondern in der Lösung, in welcher es vorgängig aus dem entsprechenden Benzodiazepin-Derivat der allgemeinen Formel Ib' hergestellt worden ist.

Zu der erwähnten, das Benzodiazepin-Derivat der allgemeinen Formel V enthaltenden Lösung kann man dann eine Aminoverbindung der allgemeinen Formel VI geben. Hierbei kann die Aminoverbindung der allgemeinen Formel VI in Form

einer Lösung oder auch ohne Lösungsmittel verwendet werden; falls es sich um eine bei Raumtemperatur gasförmige Aminoverbindung handelt (was beispielsweise für Methylamin zutrifft) kann man sie als Gas in die erwähnte, das Benzodiazepin-Derivat der allgemeinen Formel V enthaltende Lösung einleiten.

Andererseits ist es auch möglich, die Aminoverbindung der allgemeinen Formel VI vorzulegen, zweckmässigerweise in Form einer Lösung, und dann die erwähnte, das Benzodiazepin-Derivat der allgemeinen Formel V enthaltende Lösung zuzugeben.

In vielen Fällen ist es zweckmässig, die Aminoverbindung der allgemeinen Formel VI im Ueberschuss einzusetzen, und dies ist dann notwendig, wenn sie mehr als 1 Stickstoffatom enthält, welches zur Reaktion mit einer Isocyanatgruppe befähigt ist; dies ist beispielsweise beim Piperazin der Fall.

Als Lösungsmittel für den vorliegenden Verfahrensaspekt eignen sich verschiedene, unter den Reaktionsbedingungen inerte organische Lösungsmittel, beispielsweise halogenierte Kohlenwasserstoffe, wie Dichloräthan, Methylenchlorid, Chloroform, o-Dichlorbenzol usw.; Aether wie Tetrahydrofuran, Dioxan, Dimethoxyäthan, Diäthylenglykoldimethyläther usw.; oder dergleichen.

Die Umsetzung der Verbindungen der Formeln V und VI erfolgt zweckmässigerweise bei Raumtemperatur oder Temperaturen unterhalb davon. Es ist noch zu beachten, dass man dann, wenn man eine Lösung des Benzodiazepin-Derivats der allgemeinen Formel V vorlegt, die Aminoverbindung der allgemeinen Formel VI innerhalb kurzer Zeit zugeben sollte, wogegen im umgekehrten Fall, d.h. wenn man die Aminoverbindung der allgemeinen Formel VI vorlegt und dann die Lösung des Benzodiazepin-Derivats der allgemeinen Formel V

zugibt, die Geschwindigkeit der Zugabe keine wesentliche Rolle spielt.

Gemäss Verfahrensvariante g) können Benzodiazepin-Derivate der allgemeinen Formel I hergestellt werden, indem man ein Amin der allgemeinen Formel Ib mit einem Halogenid der Formel VII umsetzt. Diese Reaktion erfolgt in Gegenwart eines säurebindenden Mittels, beispielsweise einer anorganischen Base, wie Kaliumcarbonat, Natriumcarbonat usw., oder einer organischen Base, z.B. einer tertiären Aminoverbindung, wie Triäthylamin, N-Aethyl-diisopropylamin, Chinuclidin usw. Zweckmässigerweise arbeitet man bei Raumtemperatur oder unterhalb davon. Die Reaktion verläuft ziemlich langsam und dauert in der Regel mehrere Tage.

In Verbindungen der allgemeinen Formel Ib, worin A' die Gruppe (b) bedeutet, reagieren selbstverständlich beide im Molekül vorhandenen Aminogruppen mit dem Carbamoylhalogenid der Formel VII, so dass man von letzterem mindestens 2 Aequivalente einsetzen muss.

Gemäss Verfahrensvariante h) können Benzodiazepin-Derivate der allgemeinen Formel I hergestellt werden, indem man ein Amin der allgemeinen Formel Ib mit einem Isocyanat der Formel VIII umsetzt. Diese Reaktion erfolgt zweckmässigerweise in einem unter den Reaktionsbedingungen inerten organischen Lösungsmittel, beispielsweise in einem halogenierten Kohlenwasserstoff, wie Methylenchlorid, Dichloräthan, Chloroform, o-Dichlorbenzol usw.; in einem Aether, wie Tetrahydrofuran, Dioxan, Dimethoxyäthan, Diäthylenglykoldimethyläther usw.; oder dergleichen. In manchen Fällen erweist es sich als günstig, die Reaktion in Gegenwart einer katalytisch wirkenden geringen Menge einer Base durchzuführen, beispielsweise in Gegenwart einer

tertiären Aminoverbindung, wie Triäthylamin, N-Aethyl-diisopropylamin, Chinuclidin usw. Die Temperatur ist für die Umsetzung der Verbindungen der Formeln Ib und VIII nicht kritisch, und man kann demnach bei Raumtemperatur, unterhalb der Raumtemperatur oder oberhalb der Raumtemperatur arbeiten, beispielsweise bei Rückflusstemperatur.

In Verbindungen der allgemeinen Formel Ib, worin A' die Gruppe (b) bedeutet, reagieren selbstverständlich beide im Molekül vorhandenen Aminogruppen mit dem Isocyanat der Formel VIII, so dass man von letzterem mindestens 2 Aequivalente einsetzen muss.

Gemäss Verfahrensvariante i) können Benzodiazepin-Derivate der allgemeinen Formel I hergestellt werden, indem man einen Alkohol der allgemeinen Formel IX mit einer Verbindung der Formel X veräthert. Die Abgangsgruppe L in Verbindungen der Formel X kann ein Halogenatom sein, wie z.B. Chlor, Brom oder Jod, oder ein Sulfonsäurerest, wie z.B. p-Toluolsulfonyloxy, Methansulfonyloxy oder p-Brombenzolsulfonyloxy, oder ein quaternärer Ammoniumrest, wie z.B. der Trimethylammoniumrest, oder irgendeine äquivalente Abgangsgruppe. Verbindungen wie z.B. Dimethylsulfat oder Diäthylsulfat werden ebenfalls von der Formel X umfasst. Weitere geeignete Alkylierungsmittel sind beispielsweise Methyljodid, Aethylbromid, Chlordimethyläther und dergleichen.

Ueblicherweise arbeitet man in einem unter den Reaktionsbedingungen inerten organischen Lösungsmittel, beispielsweise in einem Aether, wie Tetrahydrofuran, Dioxan,

Dimethoxyäthan, und dergleichen, in Gegenwart eines säurebindenden Mittels, beispielsweise Natriumcarbonat, Kaliumcarbonat, Triäthylamin, Chinuclidin, usw. In einer bevorzugten Ausführungsform verwendet man ein basisches Lösungsmittel, wie beispielsweise Dimethylanilin, Pyridin, Triäthylamin und dergleichen.

Gemäss Verfahrensvariante j) können Benzodiazepin-Derivate der allgemeinen Formel I hergestellt werden, indem man einen Alkohol der allgemeinen Formel IX mit einem einen Rest der Formel XI liefernden Mittel umsetzt, d.h. dass man einen Alkohol der allgemeinen Formel IX zum entsprechenden Ester acyliert. Als Acylierungsmittel kommen dabei entsprechende Säurehalogenide, Säureanhydride, Säureimidazolide, gemischte Anhydride (z.B. mit Mesitylensulfonsäure, Trifluoressigsäure, und dergleichen) oder die entsprechende freie Säure in Kombination mit einem Kondensationsmittel, wie z.B. Dicyclohexylcarbodiimid, Thionyldiimidazol, 2-Chlor-1-methyl-pyridinium-jodid und dergleichen, in Frage.

Die Reaktionsbedingungen können, je nach dem zur Anwendung gelangenden Acylierungsmittel, von jedem Fachmann leicht ausgewählt werden. Beispielsweise kann man einen Alkohol der allgemeinen Formel IX mit einem Anhydrid, wie z.B. Acetanhydrid, in einem basischen organischen Lösungsmittel, wie z.B. Pyridin, zur Reaktion bringen, wobei man zweckmässigerweise bei Raumtemperatur arbeitet, man kann aber auch unterhalb oder oberhalb davon arbeiten.

Gemäss Verfahrensvariante k) kann man Benzodiazepin-Derivate der allgemeinen Formel I herstellen, indem man einen Alkohol der allgemeinen Formel IX mit einem Isocyanat der Formel XII umsetzt. Diese Reaktion erfolgt in Analogie zur Umsetzung von Verbindungen der allgemeinen Formel Ib mit Isocyanaten der allgemeinen Formel VIII, gemäss Verfahrensvariante g). In Anbetracht der geringeren

Reaktivität von Alkoholen im Vergleich zu Aminen, arbeitet man vorzugsweise bei erhöhter Temperatur, beispielsweise bei Siedetemperatur des Reaktionsgemisches und verlängert zweckmässigerweise die Reaktionszeit.

Gemäss Verfahrensvariante l) können Benzodiazepin-Derivate der allgemeinen Formel I hergestellt werden, indem man eine Iminoverbindung der allgemeinen Formel Ic zum entsprechenden Amin reduziert. Ein für diesen Verfahrensaspekt geeignetes Reduktionsmittel ist z.B. Natriumcyanoborhydrid. Arbeitet man beispielsweise in Methanol unter kontrollierten, schwach sauren Bedingungen, so wird selektiv die im Molekül vorhandene Iminogruppe zum entsprechenden Amin reduziert.

Gemäss Verfahrensvariante m) können Benzodiazepin-Derivate der allgemeinen Formel I in ihre pharmazeutisch annehmbaren Säureadditionssalze übergeführt werden. Es kommen dabei sowohl Salze mit pharmazeutisch annehmbaren anorganischen Säuren, wie beispielsweise Chlorwasserstoff, Bromwasserstoff, Schwefelsäure, Phosphorsäure und dergleichen, als auch Salze mit pharmazeutisch annehmbaren organischen Säuren, wie beispielsweise Zitronensäure, Essigsäure, Bernsteinsäure, Methansulfonsäure, p-Toluolsulfonsäure und dergleichen, in Frage. Die Herstellung solcher Salze erfolgt dabei nach allgemein üblichen und jedem Fachmann geläufigen Methoden.

Die als Ausgangsstoffe verwendeten Benzodiazepin-Derivate der allgemeinen Formel II gehören einer an sich bekannten Verbindungsklasse an, und es sind bereits diverse spezifische Vertreter dieser Verbindungsklasse in der Literatur beschrieben worden. Noch nicht spezifisch vorbeschriebene Vertreter können nach an sich bekannten, jedem Fachmann geläufigen Methoden hergestellt werden. Verschiedene der weiter unten folgenden Beispiele enthalten detaillierte Angaben betreffend die Herstellung bestimmter Verbindungen der allgemeinen Formel II.

Nitrile der allgemeinen Formel II können beispielsweise ausgehend von Nitrobenzophenon-Derivaten der Formel XIII gemäss nachfolgendem Reaktionsschema, worin $R^1$, $R^2$, $R^3$ und $R^5$ die eingangs erwähnte Bedeutung besitzen, hergestellt werden:

Reaktionsschema

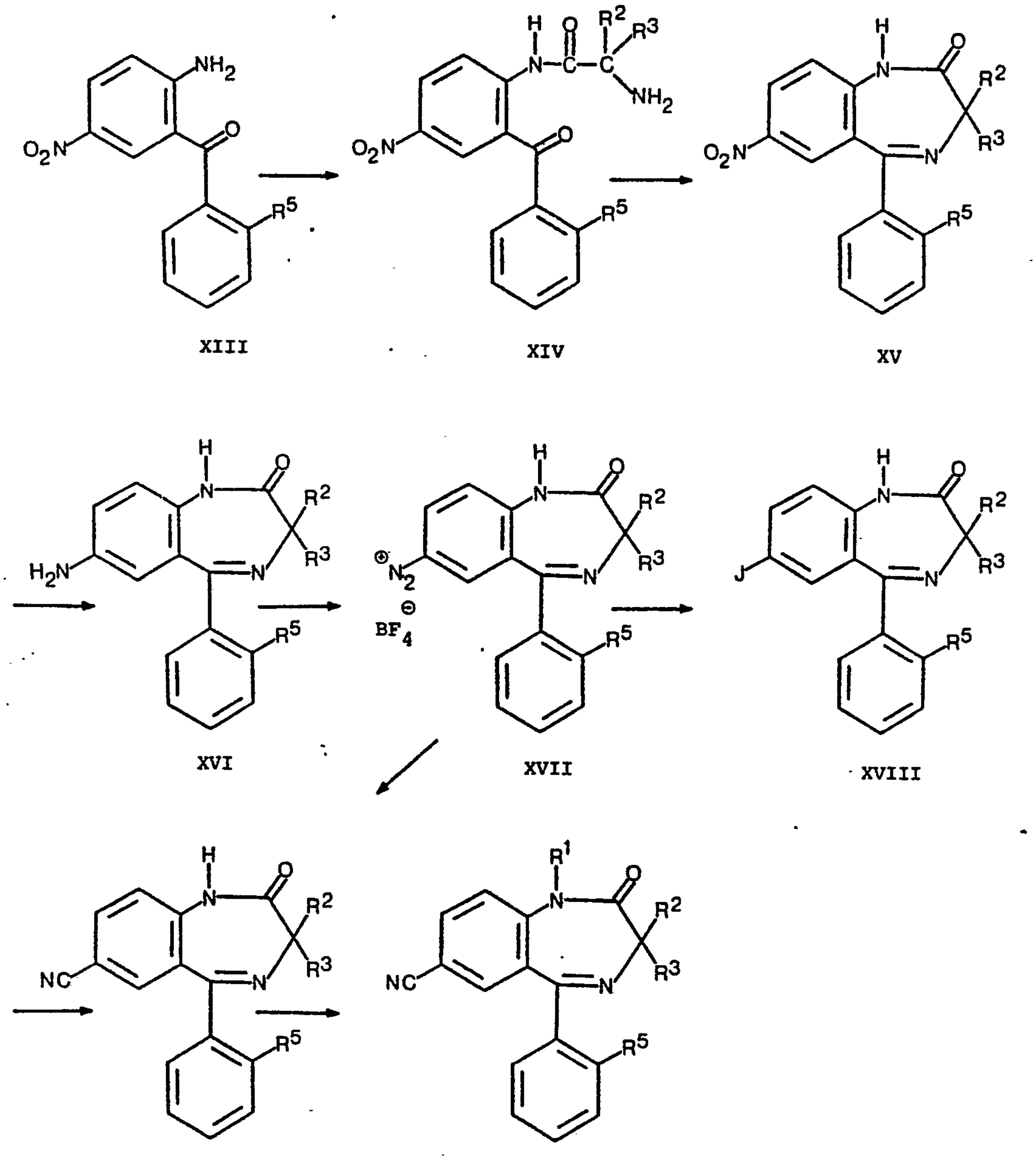

XIII XIV XV

XVI XVII XVIII

XIX II

Die als Ausgangsstoffe verwendeten Carbonylverbindungen der allgemeinen Formel III gehören einer bekannten Verbindungsklasse an und können nach allgemein bekannten und jedem Fachmann geläufigen Methoden hergestellt werden, beispielsweise ausgehend von Nitrilen der allgemeinen Formel II. Durch Behandeln solcher Nitrile mit nieder Alkyllithium in einem inerten organischen Lösungsmittel erhält man selektiv die entsprechenden 7-Alkanoyl-benzodiazepin-Derivate der allgemeinen Formel III.

Einige der weiter unten folgenden Beispiele enthalten detaillierte Angaben betreffend die Herstellung bestimmter Verbindungen der allgemeinen Formel III.

Die als Ausgangsstoffe verwendeten Isocyanate der allgemeinen Formel V können - wie bereits weiter oben erwähnt - aus entsprechenden Benzodiazepin-Derivaten der allgemeinen Formel Ib' hergestellt werden, und zwar durch Umsetzen mit Phosgen. Dabei geht man zweckmässigerweise so vor, dass man eine Lösung von Phosgen in einem unter den Reaktionsbedingungen inerten organischen Lösungsmittel vorlegt und dann unter Kühlen eine Lösung eines Benzodiazepin-Derivates der allgemeinen Formel Ib' zugibt, hierauf einige Zeit unter Rückfluss zum Sieden erhitzt, dann wieder abkühlt und schliesslich die erhaltene Lösung mit einer tertiären organischen Aminoverbindung, wie z.B. Triäthylamin, basisch oder zumindest neutral stellt. Eine so erhaltene Lösung eines Isocyanates der allgemeinen Formel V kann unter Feuchtigkeitsausschluss und in der Kälte während mehreren Stunden aufbewahrt werden. Die Lösung wird - wie bereits weiter oben ausgeführt - ohne Isolierung des darin enthaltenen Isocyanates der allgemeinen Formel V direkt weiterverarbeitet.

Die Isocyanate der allgemeinen Formel V sind ebenfalls Gegenstand der vorliegenden Erfindung.

Die als Ausgangsstoffe verwendeten Alkohole der allgemeinen Formel IX gehören einer an sich bekannten Verbindungsklasse an. Spezifisch nicht vorbeschriebene Vertreter können nach bekannten und jedem Fachmann geläufigen Methoden hergestellt werden. In einigen der weiter unten folgenden Beispiele wird die Herstellung von bestimmten Verbindungen der allgemeinen Formel IX beschrieben.

Als Ausgangsstoffe für die Herstellung von Verbindungen der allgemeinen Formel IX verwendet man zweckmässigerweise Carbonylverbindungen der allgemeinen Formel III. Durch selektive Reduktion der Carbonylgruppe erhält man die entsprechenden Alkohole der allgemeinen Formel IX. Dabei gilt es zu beachten, dass nur solche Reduktionsmittel in Frage kommen, welche die ebenfalls im Molekül vorhandene Amid- bzw. Iminogruppe nicht angreifen. Ein für diesen Zweck besonders geeignetes Reduktionsmittel ist beispielsweise Natriumborhydrid.

Ueberraschenderweise hat es sich gezeigt, dass die Benzodiazepin-Derivate der eingangs definierten allgemeinen Formel I keine oder nur sehr geringe Wirkungen auf das zentrale Nervensystem entfalten, wogegen sie ausgeprägte aldosteronantagonistische Eigenschaften aufweisen. Diese aldosteronantagonistischen Eigenschaften lassen sich, wie nachstehend erläutert, an adrenalektomierten Ratten nachweisen.

Verabreicht man adrenalektomierten Ratten Aldosteron, so beobachtet man - im Vergleich zu unbehandelten Tieren - eine ausgeprägte Verminderung der Natrium-Ausscheidung (Natrium-Retention), eine erhöhte Kalium-Ausscheidung (Kalium-Exkretion) sowie eine Verminderung des ausgeschiedenen Harnvolumens. Gibt man den Tieren vor der Behandlung mit Aldosteron Verbindungen der Formel I, so beobachtet man - im Vergleich zu nur mit Aldosteron behandelten Tieren (Kontrolltiere) - eine ausgeprägte Erhöhung der Natrium-Ausscheidung (das heisst: die durch Aldrosteron bewirkte Natrium-Retention wird antagonisiert), wogegen die Kalium-Exkretion und das Harnvolumen in geringerem Ausmass beeinflusst werden.

Der Standardversuch wird wie folgt durchgeführt:

Weibliche Holtzmann-Ratten (150-180 g) werden 70 bis 74 Stunden vor Versuchsbeginn bilateral adrenalektomiert. Nach der Operation erhalten die Tiere ein gebräuchliches Ratten-Trockenfutter und 0,9%ige Kochsalzlösung zum Trinken. 16-17 Stunden vor Versuchsbeginn wird den Tieren das Futter entzogen, wogegen sie nach wie vor ad libitum 0,9%ige Kochsalzlösung trinken können. Bei Versuchsbeginn wird den Tieren die auf Aldosteronantagonismus zu prüfende Substanz mittels einer Magensonde verabreicht. 30 Minuten später erhalten die Tiere eine subcutane Injektion von 4 mmg/kg Aldosteron. Nach weiteren 90 Minuten werden die Harnblasen der Tiere durch sorgfältiges suprapubisches Drücken entleert, worauf die Tiere ohne Nahrung und ohne Getränk einzeln in Metabolismuskäfige verbracht werden. Der Urin der Tiere wird dann während 3 Stunden gesammelt, worauf ihre Harnblasen nochmals entleert werden. Der spontan ausgeschiedene Harn und der am Schluss des Versuches durch Ausdrücken der Harnblasen erhaltene Restharn werden in graduierten Zentrifugengläsern gesammelt. Natrium- und Kalium-Konzentrationen des Harns werden mit einem Flammenphotometer bestimmt.

In der nachfolgenden Tabelle werden die Resultate dargestellt, welche mit repräsentativen Vertretern der durch die allgemeine Formel I definierten Verbindungsklasse in dem vorstehend geschilderten Versuch erhalten worden sind. Angegeben werden in dieser Tabelle für jede der darin figurierenden Verbindungen die verabreichte Dosis (in mg/kg p.o.) sowie die prozentuale Veränderung des Harnvolumens, der Natrium-Ausscheidung und der Kalium-Ausscheidung im Vergleich zu den Kontrolltieren (das heisst im Vergleich zu den nur mit Aldosteron behandelten Tieren), Ausserdem enthält die Tabelle Angaben über die akute Toxizität der untersuchten Verbindungen (DL 50 in mg/kg bei einmaliger oraler Verabreichung an Mäuse).

Toxizität und Wirkung an der adrenalektomierten Ratte

| $R^1$ | $R^2$ | $R^3$ | A | $R^5$ | $R^4$ | Dosis mg/kg p.o. | Volumen in %, | [$Na^+$] bez. auf | [$K^+$] Kontrolltiere | DL 50 mg/kg p.o. |
|---|---|---|---|---|---|---|---|---|---|---|
| Et | H | H | 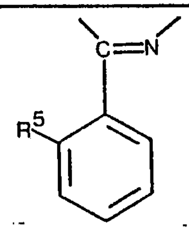 | F | $CH_3$-O-$CH_2$-O-CH-<br>$CH_3$ | 1 | 138 | 359 | 93 | 500 |
| Et | H | H | 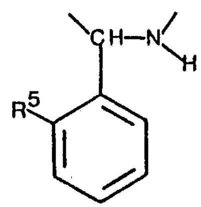 | F | HO-N=C-<br>$CH_3$ | 1 | 157 | 247 | 87 | > 5000 |
| Et | H | H | | F | HO-$(CH_2)_2$-NH-CH-<br>$CH_3$ | 1 | 150 | 306 | 93 | 4000 |
| Et | H | H | | F | $(CH_3)_2$-N-CH-<br>$CH_3$ | 1 | 147 | 252 | 90 | 500 |
| $CH_3$ | $CH_3$ | H | 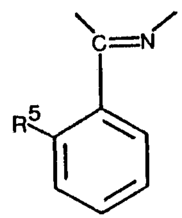 | F | $H_2$N-CH-<br>$CH_3$ | 1 | 169 | 298 | 102 | 625 |
| $CH_3$ | H | H | | H | HO-$(CH_2)_2$-NH-CO<br>-$CH_2$-NH | 1 | 130 | 224 | 82 | > 5000 |

Die Benzodiazepin-Derivate der allgemeinen Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Benzodiazepin-Derivate der allgemeinen Formel I und ihre pharmazeutisch annehmbaren Säureadditionssalze mit pharmazeutisch inerten, anorganischen oder organischen Excipientien verarbeitet werden. Als solche Excipientien kann man für Tabletten, Dragées und Hartgelatinekapseln z.B. Lactose, Maisstärke oder Derivate davon, Talk, Stearinsäure oder deren Salze etc. verwenden.

Für Weichgelatinekapseln eignen sich als Excipientien z.B. pflanzliche Oele, Wachse, Fette, halbfeste und flüssige Polyole etc.; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z.B. Wasser, Polyole, Saccharose, Invertzucker, Glukose und dergleichen.

Für Injektionslösungen eignen sich als Excipientien z.B. Wasser, Alkohole, Polyole, Glyzerin, pflanzliche Oele etc.

Für Suppositorien eignen sich als Excipientien z.B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine oder mehrere Verbindungen der allgemeinen Formel I oder pharmazeutisch annehmbare Säureadditionssalze davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt. Ein weiterer Gegenstand der vorliegenden Erfindung ist - wie eingangs erwähnt - die Verwendung von Benzodiazepin-Derivaten der allgemeinen Formel I und von pharmazeutisch annehmbaren Säureadditionssalzen davon bei der Bekämpfung bzw. Verhütung von Krankheiten, insbesondere bei der Bekämpfung bzw. Verhütung von Herzversagen, von hepatitischer Aszites, von primären Aldosteronismus und von essentieller Hypertonie. Die Dosierung kann innrehalb weiter Grenzen variieren und ist natürlich in jedem einzelnen Fall den individuellen Gegebenheiten anzupassen. Im allgemeinen dürfte bei oraler Verabreichung eine Tagesdosis von etwa 20 mg bis 1500 mg angemessen sein.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

Beispiel 1

a) Eine Lösung von 105,6 g (0,47 Mol) Carbobenzoxy-DLalanin in 830 ml Tetrahydrofuran versetzt man bei 0° tropfenweise mit 41,5 ml (68,1 g; 0,57 Mol) Thionylchlorid, rührt anschliessend das Gemisch 1 Stunde bei Raumtemperatur, versetzt mit einer Lösung von 100 g (0,38 Mol) 2-Amino-2'-fluor-5-nitrobenzophenon in 580 ml Tetrahydrofuran und rührt 24 Stunden bei Raumtemperatur. Nach Einengen des Reaktionsgemisches im Vakuum nimmt man den Rückstand in 1,8 l Methylenchlorid/Aethanol (9:1) auf, versetzt diese Lösung mit 1,0 l 1N Kaliumbicarbonatlösung und rührt während 30 Minuten. Die wässerige Phase wird abgetrennt, und mit 1,6 l Methylenchlorid ausgeschüttelt. Die vereinigten organischen Phasen werden mit Wasser gewaschen, getrocknet und eingedampft.

b) Das obige rohe Zwischenprodukt wird in 750 ml 33-proz. Bromwasserstoffsäure in Eisessig aufgenommen und 5 Stunden bei Raumtemperatur kräftig gerührt. Das Reaktionsgemisch wird anschliessend im Vakuum eingedampft und der Rückstand zwischen 3 l Wasser und 3 l Aether verteilt. Die wässrige Phase wird abgetrennt, mit 105 g festem Kaliumcarbonat neutralisiert und mit Methylenchlorid extrahiert. Die organische Phase wird nach Waschen mit Wasser getrocknet und eingedampft.

c) Das so erhaltene rohe Zwischenprodukt wird in 1,5 l Toluol und 150 ml Eisessig gelöst und im Wasserabscheider während 7 Stunden unter Rückfluss zum Sieden erhitzt. Anschliessend dampft man die Lösung im Vakuum zur Trockene ein und entfernt Spuren von Essigsäure durch azeotropes Abdampfen mit Toluol. Das rohe 5-(o-Fluorphenyl)-1,3-dihydro-3-methyl-7-nitro-2H-1,4-benzodiazepin-2-on wird ohne weitere Reinigung weiter verarbeitet.

d) 62,7 g (0,2 Mol) rohes rac-5-(o-Fluorphenyl)-1,3-dihydro-3-methyl-7-nitro-2H-1,4-benzodiazepin-2-on in 1 l konz. Salzsäure versetzt man unter Rühren portionenweise mit insgesamt 135,5 g (0,6 Mol) Zinnchlorid, rührt 2 Stunden bei Raumtemperatur, und neutralisiert das Reaktionsgemisch in der Kälte mit 10N Natronlauge. Die wässerige Phase wird mit insgesamt 8 l Methylenchlorid/Aethanol (4:1) extrahiert, die vereinigten organischen Auszüge werden mit gesättigter Kochsalzlösung gewaschen, getrocknet und eingedampft. Aus Aether erhält man 7-Amino-5-(o-fluorphenyl)-1,3-dihydro-3-methyl-2H-1,4-benzodiazepin-2-on vom Schmelzpunkt 270°.

e) Eine Lösung von 24,2 g (85,4 mMol) 7-Amino-5-(o-fluorphenyl)-1,3-dihydro-3-methyl-2H-1,4-benzodiazepin-2-on in 100 ml Wasser und 200 ml 50-proz. wässriger Borfluorwasserstoffsäure wird unter Rühren bei 0° mit einer Lösung von Natriumnitrit (93,96 mMol) in 15 ml Wasser tropfenweise so versetzt, dass die Natriumnitritlösung unterhalb der Oberfläche des Reaktionsgemisches eintritt (Additionszeit: 30 Minuten). Das Reaktionsgemisch wird anschliessend noch 1 Stunde bei 5° gerührt und dann bei -20° stehengelassen. Das auskristallisierte Material wird abfiltriert, mit kaltem Isopropanol und kaltem Aether gewaschen und im Vakuum bei Raumtemperatur getrocknet. Dabei erhält man 5-(o-Fluorphenyl)-2,3-dihydro-3-methyl-2-oxo-1H-1,4-benzodiazepin-7-diazoniumtetrafluorborat.

f) Zu einer bei Raumtemperatur und unter Lichtausschluss gerührten Lösung von 32,65 g des obigen Diazoniumsalzes und 0,918 g 18-Crown-6 in 700 ml Chloroform gibt man 33,0 g Jod zu, rührt einige Minuten und gibt 7,9 g Kaliumacetat zu. Das Gemisch wird bei Raumtemperatur während 20 Stunden gerührt, filtriert, mit 10-proz. Natriumbisulfitlösung und viermal mit Wasser gewaschen, getrocknet und eingedampft. Der Rückstand wird über 250 g Kieselgel unter Nachspülen mit Chloroform filtriert. Aus Cyclohexan erhält man

5-(o-Fluorphenyl)-1,3-dihydro-7-jod-3-methyl-2H-1,4-benzodiazepin-2-on vom Schmelzpunkt 211°.

g) 23,2 g (58,8 mMol) 5-(o-Fluorphenyl)-1,3-dihydro-7-jod-3-methyl-2H-1,4-benzodiazepin-2-on und 10 g (0,11 Mol) Kupfer(I)cyanid in 250 ml Dimethylformamid werden 2 Stunden unter Argon auf 150° erhitzt. Nach dem Erkalten gibt man 700 ml Wasser zum Reaktionsgemisch und filtriert den entstandenen Niederschlag ab. Dieser wird in 1 l Wasser/Aethylendiamin (1:1) gelöst und mit 1 l Methylenchlorid extrahiert; die abgetrennte wässrige Phase wird noch zweimal mit Methylenchlorid extrahiert. Die vereinigten organischen Auszüge werden mit Wasser gewaschen, getrocknet und eingedampft. Der Rückstand ergibt aus Cyclohexan 5-(o-Fluorphenyl)-2,3-dihydro-3-methyl-2-oxo-1H-1,4-benzodiazepin-7-carbonitril vom Schmelzpunkt 216°.

h) Zu einer bei -70° unter Argon gerührten Lösung von 12,81 g (43,68 mMol) 5-(o-Fluorphenyl)-2,3-dihydro-3-methyl-2-oxo-1H-1,4-benzodiazepin-7-carbonitril in 500 ml Tetrahydrofuran tropft man 110 ml einer 2N Lösung von Methyllithium in Aether. Nach 21 Stunden bei -70° gibt man 440 ml 2N Salzsäure zu und neutralisiert das Gemisch nach Erwärmen auf Raumtemperatur mit 135 ml 3N Natronlauge. Nach Abtrennen der wässrigen Phase dampft man die organische Phase im Vakuum bei 40° ein, nimmt den Rückstand in Methylenchlorid auf und wäscht die organische Phase mit der oben abgetrennten wässrigen Phase. Die Methylenchloridlösung wird mit Wasser gewaschen, getrocknet und eingedampft. Der Rückstand liefert aus Methylenchlorid/Cyclohexan 7-Acetyl-5-(o-fluorphenyl)-1,3-dihydro-3-methyl-2H-1,4-benzodiazepin-2-on vom Schmelzpunkt 203°.

i) Zu einer Lösung von 7,15 g (23,04 mMol) 7-Acetyl-5-(o-fluorphenyl)-1,3-dihydro-3-methyl-2H-1,4-benzodiazepin-2-on und 15 ml Methyljodid in 300 ml Aceton gibt man 7,15 g Kaliumcarbonat und rührt das Gemisch 21 Stunden bei Raumtemperatur. Das unlösliche Material wird abfiltriert und die Lösung im Vakuum eingedampft. Der Rückstand wird in Methylenchlorid aufgenommen, mit Wasser gewaschen, getrocknet und eingedampft. Aus Cyclohexan erhält man 7-Acetyl-5-(o-fluorphenyl)-1,3-dihydro-1,3-dimethyl-2H-1,4-benzodiazepin-2-on vom Schmelzpunkt 65°.

j) Eine Lösung von 4,41 g (13,60 mMol) 7-Acetyl-5-(o-fluorphenyl)-1,3-dihydro-1,3-dimethyl-2H-1,4-benzodiazepin-2-on und 4,41 g (63,46 mMol) Hydroxylaminhydrochlorid in 25 ml Pyridin wird 19 Stnden bei Raumtemperatur gerührt, anschliessend auf Eis gegossen und mit Chloroform extrahiert. Die organischen Auszüge werden nacheinander mit 2N Salzsäure und Wasser gewaschen, getrocknet und eingedampft. Nach Chromatographie des Rückstandes an 250 g Silicagel mit Chloroform/Aethanol (98:2) als Elutionsmittel erhält man 5-(o-Fluorphenyl)-1,3-dihydro-7-[1-(hydroxyimino)-äthyl]-1,3-dimethyl-2H-1,4-benzodiazepin-2-on vom Schmelzpunkt 101-103°.

## Beispiel 2

Eine Lösung von 4,1 g (12,08 mMol) 5-(o-Fluorphenyl)-1,3-dihydro-7-[1-(hydroxyimino)äthyl]-1,3-dimethyl-2H-1,4-benzodiazepin-2-on in 150 ml 2N methanolischem Ammoniak versetzt man mit 5 g Raney-Nickel-Paste und rührt die Suspension in einer Wasserstoffatomosphäre während 24 Stunden. Nach Abfiltrieren des Katalysators und Entfernen des Lösungsmittels im Vakuum erhält man 7-(1-Aminoäthyl)-5-(o-fluorphenyl)-1,3-dihydro-1,3-dimethyl-2H-1,4-benzodiazepin-2-on als Schaum vom Schmelzpunkt 48-50°.

Beispiel 3

a) Zu 13,6 g (45,9 mMol) 7-Acetyl-5-(o-fluorphenyl)-1,3-dihydro-2H-1,4-benzodiazepin-2-on in 400 ml Aceton gibt man 14,2 ml Aethylbromid und 27,2 g Kaliumcarbonat. Das Reaktionsgemisch wird während 71 Stunden bei Raumtemperatur gerührt, filtriert und im Vakuum eingedampft. Der Rückstand wird in Methylenchlorid aufgenommen, die organische Phase mit Wasser gewaschen, getrocknet und eingedampft. Aus Methylenchlorid/Cyclohexan resultiert 7-Acetyl-1-äthyl-5-(o-fluorphenyl)-1,3-dihydro-2H-1,4-benzodiazepin-2-on vom Schmelzpunkt 154-156°.

b) Eine Lösung von 13,0 g (40,1 mMol) 7-Acetyl-1-äthyl-5-(o-fluorphenyl)-1,3-dihydro-2H-1,4-benzodiazepin-2-on und 13,0 g Hydroxylamin-hydrochlorid in 65 ml Pyridin und 65 ml Aethanol wird 4 Stunden bei Raumtemperatur stehengelassen. Nach Aufnehmen in Methylenchlorid wird mit 2N Salzsäure und Wasser gewaschen, getrocknet und eingedampft. Spuren von Pyridin entfernt man durch azeotropes Eindampfen mit Toluol. Der Rückstand ergibt aus Methylenchlorid/Hexan 1-Aethyl-5-(o-fluorphenyl)-1,3-dihydro-7-[1-(hydroxyimino)-äthyl]-2H-1,4-benzodiazepin-2-on vom Schmelzpunkt 236-238°.

Beispiel 4

Eine Lösung von 1 g (2,95 mMol) 1-Aethyl-5-(o-fluorphenyl)-1,3-dihydro-7-[1-(hydroxyimino)äthyl]-2H-1,4-benzodiazepin-2-on in 150 ml Aethanol wird mit 1,0 g Raney-Nickel-Paste während 21 Stunden in einer Wasserstoffatmosphäre bei 50° gerührt. Nach Filtrieren über Digalite und Eindampfen im Vakuum erhält man 7-(1-Aminoäthyl)-1-äthyl-5-(o-fluorphenyl)-1,3-dihydro-2H-1,4-benzodiazepin-2-on vom Schmelzpunkt 53-55°.

Beispiel 5

Zu einer bei Raumtemperatur gerührten Suspension von 3,0 g (7,85 mMol) 1-Aethyl-5-(o-fluorphenyl)-1,3-dihydro-7-[1-(hydroxyimino)äthyl]-2H-1,4-benzodiazepin-2-on in 30 ml Methanol gibt man 372 mg (5,91 mMol) Natriumcyanoborhydrid und 1 Tropfen 1-proz. äthanolische Bromkresollösung. Anschliessend tropft man im Verlauf von 30 Minuten 30 ml 2N methanolische Salzsäure so zu, dass die gelbe Färbung des Reaktionsgemisches erhalten bleibt. Man rührt bei Raumtemperatur 48 Stunden, wobei nach 24 Stunden noch 372 mg Natriumcyanborhydrid zugegeben werden. Das Reaktionsgemisch wird im Vakuum eingeengt und der Rückstand mit 6N Natronlauge auf pH 10 gestellt. Nach Extrahieren mit Chloroform wird die organische Phase mit Wasser gewaschen, getrocknet und eingedampft. Der Rückstand ergibt aus Aether 1-Aethyl-5-(o-fluorphenyl)-1,3,4,5-tetrahydro-7-[1-(hydroxyimino)äthyl]-2H-1,4-benzodiazepin-2-on vom Schmelzpunkt 237-239°.

Beispiel 6

Zu einer bei Raumtemperatur gerührten Lösung von 7,98 ml Aethanolamin in 140 ml Methanol und 9,0 ml 5N methanolischer Salzsäure werden 7,0 g (21,7 mMol) 7-Acetyl-1-äthyl-5-(o-fluorphenyl)-1,3-dihydro-2H-1,4-benzodiazepin-2-on, gefolgt von 959 mg Natriumcyanborhydrid, gegeben. Das Gemisch wird unter Argon bei Raumtemperatur während 168 Stunden gerührt, wobei man nach 48 Stunden und 144 Stunden mit je 959 mg Natriumcyanoborhydrid versetzt. Nach Entfernen des Lösungsmittels im Vakuum wird der Rückstand mit 6N Natronlauge auf pH 10 gestellt und mit Chloroform extrahiert. Nach Waschen der organischen Phase mit Wasser, Trocknen und Eindampfen wird der Rückstand an 200 g Silicagel mit Chloroform als Elutionsmittel chromatographiert. Es resultiert 1-Aethyl-5-(o-fluorphenyl)-1,3,4,5-tetrahydro-7-/1-[(2-hydroxyäthyl)amino]äthyl/-2H-1,4-benzodiazepin-2-on vom Schmelzpunkt 49-50°.

## Beispiel 7

a) Zu einer bei Raumtemperatur gerührten Lösung von 9,1 ml Dimethylamin in 140 ml Methanol und 0,1 ml 5N methanolischer Salzsäure gibt man 7,0 g (21,7 mMol) 7-Acetyl-1-äthyl-5-(o-fluorphenyl)-1,3-dihydro-2H-1,4-benzodiazepin-2-on, gefolgt von 0,24 g Natriumcyanborhydrid. Das Gemisch wird während 14 Tagen bei Raumtemperatur gerührt, wobei nach 2 bzw. 7 Tagen je 924 mg Natriumcyanoborhydrid zugegeben werden. Das Lösungsmittel wird im Vakuum entfernt; der Rückstand wird mit 10N Natronlauge auf pH 10 eingestellt und in Chloroform aufgenommen. Die organische Phase wird mit Wasser gewaschen, getrocknet und eingedampft. Den Rückstand chromatographiert man an 200 g Kieselgel mit Chloroform als Elutionsmittel und erhält 7-[1-(Dimethylamino)äthyl]-1-äthyl-5-(o-fluorphenyl)-1,3,4,5-tetrahydro-2H-1,4-benzodiazepin-2-on als Oel.

b) Eine Lösung von 2,32 g des so erhaltenen Produktes in 50 ml Aether wird in der Kälte mit gasförmigem Chlorwasserstoff behandelt. Das ausgefallene Material wird abfiltriert, mit Aether gewaschen und im Vakuum getrocknet. Man erhält 7-[1-(Dimethylamino)äthyl]-1-äthyl-5-(o-fluorphenyl)-1,3,-4,5-tetrahydro-2H-1,4-benzodiazepin-2-on-dihydrochlorid vom Schmelzpunkt 160°.

## Beispiel 8

a) 12,7 g (39,2 mMol) 7-Acetyl-1-äthyl-5-(o-fluorphenyl)-1,3-dihydro-2H-1,4-benzodiazepin-2-on in 240 ml Methanol werden in der Kälte unter Rühren mit 743 mg (19,6 mMol) Natriumborhydrid versetzt. Das Gemisch wird anschliessend 17 Stunden bei Raumtemperatur gerührt, mit Wasser und dann mit 2N Schwefelsäure versetzt und mit Methylenchlorid ausgeschüttelt. Die organische Phase wird mit Wasser gewaschen, getrocknet und eingedampft. Der Rückstand wird an 500 g Silicagel chromatographiert, mit Essigester/Toluol (1:1)

als Elutionsmittel, und ergibt aus Cyclohexan 1-Aethyl-5-(o-fluorphenyl)-1,3-dihydro-7-(1-hydroxyäthyl)-2H-1,4-benzodiazepin-2-on vom Schmelzpunkt 55-57°.

b) Eine Lösung von 3,0 g (9,2 mMol) 1-Aethyl-5-(o-fluorphenyl)-1,3-dihydro-7-(1-hydroxyäthyl)-2H-1,4-benzodiazepin-2-on in 10 ml Pyridin und 10 ml Acetanhydrid wird bei Raumtemperatur über Nacht stehengelassen und im Vakuum eingeengt. Der Rückstand wird in Methylenchlorid aufgenommen; die organische Phase wird mit Wasser gewaschen, getrocknet und eingedampft. Dabei resultiert 1-[1-Aethyl-5-(o-fluorphenyk)-2,3-dihydro-2-oxo-1H-1,4-benzodiazepin-7-yl]äthylacetat als dünnschichtchromatographisch einheitliches Oel.

## Beispiel 9

a) Ein Gemisch aus 3 g (9,2 mMol) 1-Aethyl-5-(o-fluorphenyl)-1,3-dihydro-7-(1-hydroxyäthyl)-2H-1,4-benzodiazepin-2-on, 4,7 ml Dimethylanilin und 1,4 ml Chlordimethyläther unter Argon wird 1 Stunde bei 50° und 4 Tage bei Raumtemperatur stehengelassen. Bei 0° stellt man mit 1N Salzsäure sauer, extrahiert mit Chloroform/Aethanol (9:1) und wäscht die organische Phase mit Wasser. Nach Trocknen und Eindampfen wird der Rückstand an 100 g Kieselgel mit Chloroform als Elutionsmittel chromatographiert. Es resultiert 1-Aethyl-5-(o-fluorphenyl)-1,3-dihydro-7-[1-(methoxymethoxy)äthyl]-2H-1,4-benzodiazepin-2-on als Oel.

b) In eine Lösung des so erhaltenen Produktes in 100 ml Aether wird trockener gasförmiger Chlorwasserstoff eingeleitet. Das ausgefallene Material wird abfiltriert, mit Aether gewaschen und im Vakuum getrocknet. Man erhält 1-Aethyl-5-(o-fluorphenyl)-1,3-dihydro-7-[1-(methoxymethoxy)äthyl]-2H-1,4-benzodiazepin-2-on-hydrochlorid.

Beispiel 10

Eine Lösung von 2,0 g (6,12 mMol) 1-Aethyl-5-(o-fluorphenyl)-1,3-dihydro-7-(1-hydroxyäthyl)-2H-1,4-benzodiazepin-2-on und 0,5 ml N-Phenyl-isocyanat in 35 ml Benzol wird 50 Stunden unter Rückfluss zum Sieden erhitzt, wobei man nach 20 und 35 Stunden mit je 1 ml Triäthylamin versetzt. Das Reaktionsgemisch wird im Vakuum eingedampft; der Rückstand wird mit Wasser verdünnt und mit Chloroform/Aethanol (9:1) extrahiert. Die organische Phase wird mit Wasser gewaschen, getrocknet und eingedampft. Der Rückstand wird an 100 g Silicagel mit Chloroform/Aethanol (98:2) als Elutionsmittel chromatographiert und ergibt aus Cyclohexan 1-[1-Aethyl-5-(o-fluorphenyl)-2,3-dihydro-2-oxo-1H-1,4-benzodiazepin-7-yl]-äthyl-carboxanilid vom Schmelzpunkt 83-85°.

Beispiel 11

Aus 2 g (6,12 mMol) 1-Aethyl-5-(o-fluorphenyl)-1,3-dihydro-7-(1-hydroxyäthyl)-2H-1,4-benzodiazepin-2-on und 0,7 ml N-Butyl-isocyanat erhält man in Analogie zu Beispiel 10, nach Umkristallisieren aus Cyclohexan 1-[1-Aethyl-5-(o-fluorphenyl)-2,3-dihydro-2-oxo-1H-1,4-benzodiazepin-7-yl]äthyl-butylcarbamat vom Schmelzpunkt 48-50°.

Beispiel 12

a) 42,3 g (0,41 Mol) α-Amino-isobuttersäure und 50 ml (0,68 Mol) Thionylchlorid in 400 ml Tetrahydrofuran werden während 24 Stunden bei Raumtemperatur kräftig gerührt, mit 100 g (0,38 Mol) 2-Amino-5-nitro-2'-fluorbenzophenon versetzt und während weiteren 108 Stunden bei Raumtemperatur gerührt. Das Reaktionsgemisch wird im Vakuum eingeengt, auf 500 ml 10-proz., eisgekühlte Natriumbicarbonatlösung gegossen und mit Methylenchlorid extrahiert. Nach Waschen der organischen Phase mit Natriumbicarbonatlösung und

Wasser wird das Lösungsmittel im Vakuum entfernt und das verbleibende, rohe 2-Amino-2'-(o-fluorbenzoyl)-2-methyl-4'-nitropropionanilid direkt weiterverarbeitet.

b) 184 g des obigen Rohproduktes in 1 l Toluol und 100 ml Eisessig werden während 21 Stunden in einem Wasserabscheider zum Rückfluss erhitzt und anschliessend im Vakuum zur Trockne eingedampft. Der Rückstand wird in 500 ml siedendem Aether aufgeschlämmt und abfiltriert. Es resultiert 5-(o-Fluorphenyl)-1,3-dihydro-3,3-dimethyl-7-nitro-2H-1,4-benzodiazepin-2-on vom Schmelzpunkt 241°.

c) Aus 122,3 g (0,37 Mol) 5-(o-Fluorphenyl)-1,3-dihydro-3,3-dimethyl-7-nitro-2H-1,4-benzodiazepin-2-on erhält man in Analogie zu Absatz d) von Beispiel 1 nach Umkristallisieren aus Essigester 7-Amino-5-(o- fluorphenyl)-1,3-dihydro-3,3-dimethyl-2H-1,4-benzodiazepin-2-on vom Schmelzpunkt 229-231°.

d) Aus 56,62 g (0,19 Mol) 7-Amino-5-(o-fluorphenyl)-1,3-dihydro-3,3-dimethyl-2H-1,4-benzodiazepin-2-on erhält man in Analogie zu Absatz e) von Beispiel 1 5-(o-Fluorphenyl)-2,3-dihydro-3,3-dimethyl-2-oxo-1H-1,4-benzodiazepin-7-diazoniumtetrafluorborat vom Schmelzpunkt 203°.

e) Aus 90 g (0,185 Mol) 5-(o-Fluorphenyl)-2,3-dihydro-3,3-dimethyl-2-oxo-1H-1,4-benzodiazepin-7-diazoniumtetrafluorborat erhält man in Analogie zu Absatz f) von Beispiel 1 nach Umkristallisieren aus Cyclohexan 5-(o-Fluorphenyl)-1,3-dihydro-7-jod-3,3-dimethyl-2H-1,4-benzodiazepin-2-on vom Schmelzpunkt 202°.

f) Aus 66,3 g (0,162 Mol) 5-(o-Fluorphenyl)-1,3-dihydro-7-jod-3,3-dimethyl-2H-1,4-benzodiazepin-2-on erhält man in Analogie zu Absatz g) von Beispiel 1 nach Umkristallisieren aus Cyclohexan 5-(o-Fluorphenyl)-2,3-dihydro-3,3-dimethyl-2-oxo-1H-1,4-benzodiazepin-7-carbonitril vom Schmelzpunkt 212°.

g) Aus 33,74 g (0,169 Mol) 5-(o-Fluorphenyl)-2,3-dihydro-3,3-dimethyl-2-oxo-1H-1,4-benzodiazepin-7-carbonitril erhält man in Analogie zu Absatz h) von Beispiel 1 7-Acetyl-5-(o-fluorphenyl)-1,3-dihydro-3,3-dimethyl-2H-1,4-benzodiazepin-2-on vom Schmelzpunkt 206°.

h) Aus 9,20 g (25,28 mMol) 7-Acetyl-5-(o-fluorphenyl)-1,3-dihydro-3,3-dimethyl-2H-1,4-benzodiazepin-2-on erhält man in Analogie zu Absatz i) von Beispiel 1 nach Umkristallisieren aus Cyclohexan 7-Acetyl-5-(o-fluorphenyl)-1,3-dihydro-1,3,3-trimethyl-2H-1,4-benzodiazepin-2-on vom Schmelzpunkt 59°.

i) Eine Lösung von 13,66 g (40,37 mMol) 7-Acetyl-5-(o-fluorphenyl)-1,3-dihydro-1,3,3-trimethyl-2H-1,4-benzodiazepin-2-on und 13,6 g (0,196 Mol) Hydroxylamin-hydrochlorid in 50 ml Pyridin wird 18 Stunden bei Raumtemperatur gerührt, dann auf Eis gegossen und mit Methylenchlorid/Aethanol (9:1) extrahiert. Die organische Phase wird nacheinander mit 3N Salzsäure und Wasser gewaschen, getrocknet und eingedampft. Den Rückstand chromatographiert man an 150 g Silicagel mit Methylenchlorid als Elutionsmittel und erhält aus Aether 5-(o-Fluorphenyl)-1,3-dihydro-7-[1-(hydroxyimino)äthyl]-1,3,3-trimethyl-2H-1,4-benzodiazepin-2-on vom Schmelzpunkt 199°.

Beispiel 13

Zu einer Lösung von 2,22 g (6,28 mMol) 5-(o-Fluorphenyl)-1,3-dihydro-7-[1-(hydroxyimino)äthyl]-1,3,3-trimethyl-2H-1,4-benzodiazepin-2-on in 100 ml 2N methanolischem Ammoniak gibt man 2,5 g Raney-Nickel-Paste und rührt das Gemisch während 24 Stunden bei Raumtemperatur in einer Wasserstoffatmosphäre. Der Katalysator wird abfiltriert; und das Lösungsmittel im Vakuum eingeengt. Der Rückstand

ergibt nach Filtrieren über 50 g Kieselgel mit Chloroform als Elutionsmittel 7-(1-Aminoäthyl)-5-(o-fluorphenyl)-1,3-dihydro-1,3,3-trimethyl-2H-1,4-benzodiazepin-2-on als Schaum vom Schmelzpunkt 50-51°.

## Beispiel 14

a) 2,3 g (6,8 mMol) 7-(1-Aminoäthyl)-5-(o-fluorphenyl)-1,3-dihydro-1,3,3-trimethyl-2H-1,4-benzodiazepin-2-on in 150 ml Dichloräthan werden unter Rückfluss in Lösung gebracht. Diese Lösung tropft man unter Eiskühlen und Rühren so zu einer Lösung von 2 g Phosgen in 70 ml Dichloräthan, dass die Temperatur des Reaktionsgemisches 20° nicht übersteigt. Anschliessend wird das Reaktionsgemisch 1 Stunde unter Rückfluss zum Sieden erhitzt. Anschliessend destilliert man 100 ml Dichloräthan ab und versetzt mit 50 ml frischem Lösungsmittel. Ein Argon-Strom wird unter Eiskühlen solange in die Reaktionslösung eingeleitet, bis diese eine Temperatur von 10° erreicht hat. Man erhält so eine Lösung von 7-(1-Aminoäthyl)-5-(o-fluorphenyl)-1,3-dihydro-1,3,3-trimethyl-2H-2-oxo-1,4-benzodiazepin-7-yl]-isocyanat.

b) Eine Suspension von 2,16 g Natriumcarbonat in 1,25 ml Aethanolamin und 50 ml Acetonitril wird auf einmal zur obigen Lösung des Isocyanates gegeben und 20 Stunden bei Raumtemperatur gerührt. Das Lösungsmittel wird anschliessend im Vakuum entfernt und der Rückstand in Methylenchlorid/Aethanol (4:1) aufgenommen. Die organische Phase wird mit Wasser gewaschen, getrocknet und eingedampft. Den Rückstand filtriert man über 30 g Silicagel unter Nachspülen mit Chloroform und erhält 1-/1-[5-(o-Fluorphenyl)-2,3-dihydro-1,3,3-trimethyl-2-oxo-1H-1,4-benzodiazepin-7-yl]äthyl/-3-(2-hydroxyäthyl)harnstoff als Schaum vom Schmelzpunkt 83°.

Beispiel 15

a) Aus 7,53 g (22,25 mMol) 7-Acetyl-5-(o-fluorphenyl)-1,3-dihydro-1,3,3-trimethyl-2H-1,4-benzodiazepin-2-on erhält man in Analogie zu Beispiel 8a, 5-(o-Fluorphenyl)-1,3-dihydro-7-(1-hydroxyäthyl)-1,3,3-trimethyl-2H-1,4-benzodiazepin-2-on vom Schmelzpunkt 68°.

b) Eine Lösung von 2,5 g (7,34 mMol) 5-(o-Fluorphenyl)-1,3-dihydro-7-(1-hydroxyäthyl)-1,3,3-trimethyl-2H-1,4-benzodiazepin-2-on in 10 ml Essigsäureanhydrid und 10 ml Pyridin wird während 24 Stunden bei Raumtemperatur stehengelassen, dann im Vakuum eingeengt. Der Rückstand wird in Chloroform aufgenommen; die organische Phase wird nacheinander mit 2N Salzsäure, 2N Natriumbicarbonatlösung und Wasser gewaschen, getrocknet und eingedampft. Der Rückstand wird an 50 g Kieselgel mit Chloroform als Elutionsmittel chromatographiert. Es resultiert 1-[5-(o-Fluorphenyl)-2,3-dihydro-1,3,3-trimethyl-2-oxo-1H-1,4-benzodiazepin-7-yl]-äthylacetat als Oel.

Beispiel 16

a) Zu einer eisgekühlten Lösung von 11 g (38,8 mMol) 7-Amino-5-(o-fluorphenyl)-1,3-dihydro-1-methyl-2H-1,4-benzodiazepin-2-on in 50 ml Wasser und 100 ml 50-proz. wässriger Borfluorwasserstoffsäure gibt man unter Rühren eine Lösung von 2,9 g (42 mMol) Natriumnitrit in 7 ml Wasser so zu, dass die Natriumnitritlösung unterhalb der Oberfläche des Reaktionsgemisches eintritt (Additionszeit ca. 15 Minuten). Das Reaktionsgemisch wird weitere 30 Minuten bei 5° gerührt, dann bei -24° während 22 Stunden stehengelassen. Die ausgefallenen Kristalle werden abfiltriert, mit Isopropanol und Aether gewaschen und bei 14 Torr und Raumtemperatur getrocknet. Es resultiert

5-(o-Fluorphenyl)-2,3-dihydro-1-methyl-2-oxo-1H-1,4-benzodiazepin-7-diazoniumtetrafluorborat (1:2) vom Schmelzpunkt 120-125°.

b) Zu einer Lösung von 1,675 g (3,6 mMol) des unter a) hergestellten Diazoniumsalzes und 33 mg (0,12 mMol) 18-Crown-6 in 25 ml N-Methyl-pyrrolidon gibt man 1,34 g (15 mMol) Kupfer(I)cyanid und 0,98 g (10 mMol) Kaliumacetat und rührt das Gemisch 4 Stunden unter Lichtausschluss bei Raumtemperatur. Nach Zugabe von 60 ml Wasser wird der Rückstand abfiltriert und in 100 ml Aethylendiamin/Wasser (1:1) und 150 ml Dichlormethan aufgenommen. Die abgetrennte Amin-Wasser-Phase extrahiert man noch mit 150 ml Dichlormethan. Die organischen Phasen werden mit Wasser gewaschen, getrocknet und eingedampft. Der Rückstand wird an 50 g Kieselgel mit Chloroform als Elutionsmittel chromatographiert, wobei man aus Cyclohexan 5-(o-Fluorphenyl)-2,3-dihydro-1-methyl-2-oxo-1H-1,4-benzodiazepin-7-carbonitril vom Schmelzpunkt 177° erhält.

c) 36,5 g (0,08 Mol) 5-(o-Fluorphenyl)-2,3-dihydro-1-methyl-2-oxo-1H-1,4-benzodiazepin-7-carbonitril in 2700 ml Methanol und 270 ml Ammoniak gelöst, werden in einem Autoklav mit 36,5 g Raney-Nickel bei 50° und 3,5 atm hydriert. Der Katalysator wird abfiltriert und das Lösungsmittel im Vakuum entfernt. Es resultiert 7-(Aminomethyl)-5-(o-fluorphenyl)-1,3-dihydro-1-methyl-2H-1,4-benzodiazepin-2-on als Schaum.

## Beispiel 17

Aus 7 g (23,5 mMol) 7-(Aminoäthyl)-5-(o-fluorphenyl)-1,3-dihydro-1-methyl-2H-1,4-benzodiazepin-2-on erhält man in Analogie zu Beispiel 14,über das [5-(o-Fluorphenyl)-2,3-dihydro-1-methyl-2-oxo-1H-1,4-benzodiazepin-7-yl]-

methyl/isocyanat, den gewünschten 1-/[5-(o-Fluorphenyl)-2,3-dihydro-1-methyl-2-oxo-1H-1,4-benzodiazepin-7-yl]-methyl/-3-(2-hydroxyäthyl)harnstoff als Schaum vom Schmelzpunkt 125°.

Beispiel 18

Aus 7 g (22,6 mMol) 7-(Aminoäthyl)-5-(o-fluorphenyl)-1,3-dihydro-1-methyl-2H-1,4-benzodiazepin-2-on und Pyrrolidin, an Stelle von Aethanolamin, erhält man in Analogie zu Beispiel 14, über das /[5-(o-Fluorphenyl)-2,3-dihydro-1-methyl-2-oxo-1H-1,4-benzodiazepin-7-yl]methyl/-1-isocyanat, den gewünschten N-/[5-(o-Fluorphenyl)-2,3-dihydro-1-methyl-2-oxo-1H-1,4-benzodiazepin-7-yl]methyl/-1-pyrrolidincarboxamid als Schaum vom Schmelzpunkt 145°.

Beispiel 19

Aus 7 g (22,6 mMol) 7-(Aminoäthyl)-5-(o-fluorphenyl)-1,3-dihydro-1-methyl-2H-1,4-benzodiazepin-2-on und Dimethylamin, anstelle von Aethanolamin, erhält man in Analogie zu Beispiel 14, über das /[5-(o-Fluorphenyl)-2,3-dihydro-1-methyl-2-oxo-1H-1,4-benzodiazepin-7-yl]methyl/-isocyanat, den gewünschten 3-/[5-(o-Fluorphenyl)-2,3-dihydro-1-methyl-2-oxo-1H-1,4-benzodiazepin-7-yl]methyl/-1,1-dimethylharnstoff als Schaum vom Schmelzpunkt 105°.

Beispiel 20

Eine Lösung von 6,0 g (20,43 mMol) 7-Cyano-1,3-dihydro-5-(o-fluorphenyl)-1-methyl-2H-1,4-benzodiazepin-2-on in 150 ml 2N ammoniakalischem Methanol wird mit 6,0 g Raney-Nickel-Paste 3 Tage in einer Wasserstoffatmosphäre bei 50-60° gehalten. Nach Abfiltrieren des Katalysators wird das Lösungsmittel im Vakuum entfernt; der Rückstand, in 50 ml Methylenchlorid gelöst, wird mit 2,0 ml Butylisocyanat versetzt, 4 Tage bei Raumtemperatur stehengelassen und mit Methylenchlorid verdünnt. Die organische Phase wird mit Wasser gewaschen, getrocknet und eingedampft. Den Rückstand chromatographiert man an 100 g Kieselgel mit Chloroform als Elutionsmittel. Aus Essigester resultiert 1-Butyl-3-/[4-(butylcarbamoyl)-5-(o-fluorphenyl)-2,3,4,5-tetrahydro-1-methyl-2-oxo-1H-1,4-benzodiazepin-7-yl]methyl/harnstoff vom Schmelzpunkt 172°.

## Beispiel 21

Aus 1,3-Dihydro-7-nitro-5-phenyl-2H-1,4-benzodiazepin-2-on erhält man in Analogie zu den Absätzen d), e), f) und g) von Beispiel 1 2,3-Dihydro-2-oxo-5-phenyl-1H-1,4-benzodiazepin-7-carbonitril, welches nach Behandeln mit Methyljodid und Kaliumcarbonat in Analogie zu Absatz i) von Beispiel 1 2,3-Dihydro-1-methyl-2-oxo-5-phenyl-1H-1,4-benzodiazepin-7-carbonitril ergibt. Hydrieren mit Raney-Nickel unter denselben Bedingungen, wie in Beispiel 16c angegeben, ergibt nach Umkristallisieren aus Aether 7-(Aminomethyl)-1,3-dihydro-1-methyl-5-phenyl-2H-1,4-benzodiazepin-2-on vom Schmelzpunkt 230° (Zers.).

## Beispiel 22

Aus 7 g (25 mMol) 7-(Aminomethyl)-1,3-dihydro-1-methyl-5-phenyl-2H-1,4-benzodiazepin-2-on erhält man in Analogie zu Beispiel 14, über das [(2,3-Dihydro-1-methyl-2-oxo-5-phenyl-1H-1,4-benzodiazepin-7-yl)methyl]isocyanat, nach Umkristallisieren aus Aethanol den gewünschten 1-[(2,3-Dihydro-1-methyl-2-oxo-5-phenyl-1H- 1,4-benzodiazepin-7-yl)methyl]-3-(2-hydroxyäthyl)harnstoff vom Schmelzpunkt 201°.

## Beispiel A

1-Aethyl-5-(o-fluorphenyl)-2,3-dihydro-7-[1-(methoxymethoxy)äthyl]-2H-1,4-benzodiazepin-2-on kann wie folgt als Wirkstoff zur Herstellung von pharmazeutischen Präparaten verwendet werden:

| a) Tabletten | 1 Tablette enthält |
|---|---|
| Wirkstoff | 200 mg |
| mikrokristalline Cellulose | 155mg |
| Maisstärke | 25 mg |
| Talk | 25 mg |
| Hydroxypropylmethylcellulose | 20 mg |
| | 425 mg |
| | ====== |

Der Wirkstoff wird mit der Hälfte der mikrokristallinen Cellulose gemischt und mit einer 10-proz. Lösung von Hydroxypropylmethylcellulose in einem Gemisch von Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

| b) Kapseln | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |
| | ======== |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Grösse abgefüllt.

Patentansprüche

1. Benzodiazepin-Derivate der allgemeinen Formel

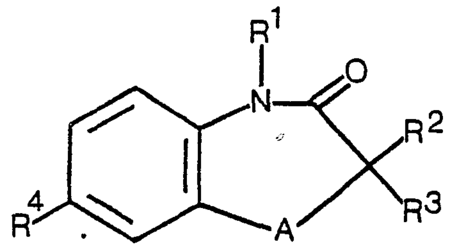 I

worin A die Gruppe

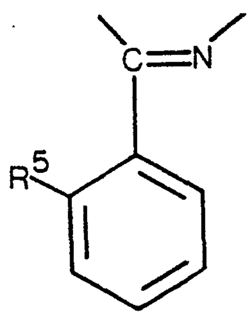 (a), 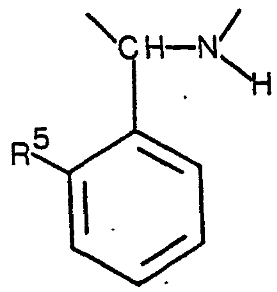 (b) oder 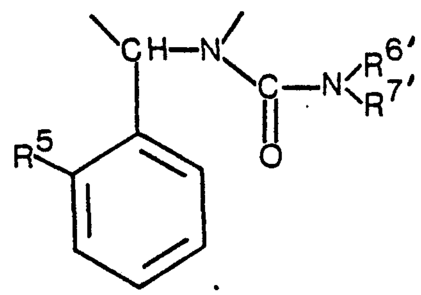 (c),

$R^1$ niederes Alkyl, $R^2$ und $R^3$ je Wasserstoff oder niederes Alkyl, $R^4$ die Gruppe

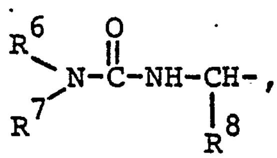 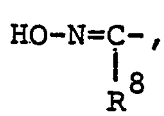 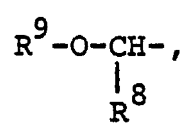 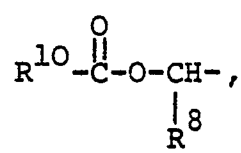

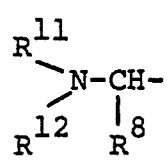 oder 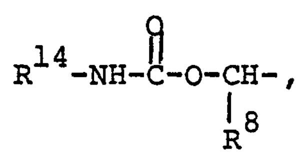

$R^5$ Wasserstoff oder Halogen, $R^8$ Wasserstoff oder niederes Alkyl, $R^9$ niederes Alkyl oder niederes Alkoxyalkyl, $R^{10}$ niederes Alkyl, $R^{11}$ Wasserstoff, niederes Alkyl oder niederes Hydroxyalkyl, $R^{12}$ Wasserstoff oder niederes Alkyl und $R^{14}$ niederes Alkyl oder Aryl

bedeuten, und entweder $R^6$ Wasserstoff oder niederes Alkyl und $R^7$ niederes Alkyl oder niederes Hydroxyalkyl bedeuten oder $R^6$ und $R^7$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13}$ als Ringglied enthalten kann, wobei $R^{13}$ Wasserstoff oder niederes Alkyl bedeutet, und entweder $R^{6'}$ Wasserstoff oder niederes Alkyl und $R^{7'}$ niederes Alkyl bedeuten oder $R^{6'}$ und $R^{7'}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13'}$ als Ringglied enthalten kann, wobei $R^{13'}$ niederes Alkyl bedeutet, mit der Massgabe, dass $R^4$ die Gruppe $R^{6'}R^{7'}N-CO-NH-CH(R^8)-$ bedeutet, wenn A die Gruppe (c) bedeutet,
und pharmazeutisch annehmbare Säureadditionssalze davon.

2. Verbindungen gemäss Anspruch 1, dadurch gekennzeichnet, das entweder $R^1$ und $R^2$ beide Methyl oder $R^1$ Aethyl und $R^2$ Wasserstoff bedeuten, wenn $R^3$ Wasserstoff, $R^4$ die Gruppe $HO-N=C(CH_3)-$, A die Gruppe (a) und $R^5$ Fluor bedeuten.

3. Verbindungen gemäss Anspruch 1, dadurch gekennzeichnet, dass $R^2$ und $R^3$ beide niederes Alkyl bedeuten, wenn $R^4$ die Gruppe $HO-N=C(R^8)-$ und A die Gruppe (a) bedeuten.

4. Verbindungen gemäss einem der Ansprüche 1 bis 3, dadurch gekennzeichnet, dass A die Gruppe (a) oder (b), $R^1$ Methyl oder Aethyl, $R^2$ und $R^3$ Wasserstoff oder Methyl, $R^4$ die Gruppe $R^6R^7N-CO-NH-CH(R^8)-$, $HO-N=C(R^8)-$, $R^9-O-CH(R^8)-$ oder $R^{11}R^{12}N-CH(R^8)-$, $R^5$ Wasserstoff oder Fluor und $R^8$ Wasserstoff oder Methyl bedeuten.

5. 1-Aethyl-5-(o-fluorphenyl)-1,3-dihydro-7-[1-(methoxymethoxy)äthyl]-2H-1,4-benzodiazepin-2-on.

6. 1-Aethyl-5-(o-fluorphenyl)-1,3,4,5-tetrahydro-7-[1-(hydroxyimino)äthyl]-2H-1,4-benzodiazepin-2-on.

7. 1-Aethyl-5-(o-fluorphenyl)-1,3,4,5-tetrahydro-7-/1-[(2-hydroxyäthyl)amino]äthyl/-2H-1,4-benzodiazepin-2-on.

8. 7-[1-(Dimethylamino)äthyl]-1-äthyl-5-(o-fluorphenyl)-1,3,4,5-tetrahydro-2H-1,4-benzodiazepin-2-on.

9. 7-(1-Aminoäthyl)-5-(o-fluorphenyl)-1,3-dihydro-1,3-dimethyl-2H-1,4-benzodiazepin-2-on.

10. 1-[(2,3-Dihydro-1-methyl-2-oxo-5-phenyl-1H-1,4-benzodiazepin-7-yl)methyl]-3-(2-hydroxyäthyl)harnstoff.

11. Verbindungen der allgemeinen Formel

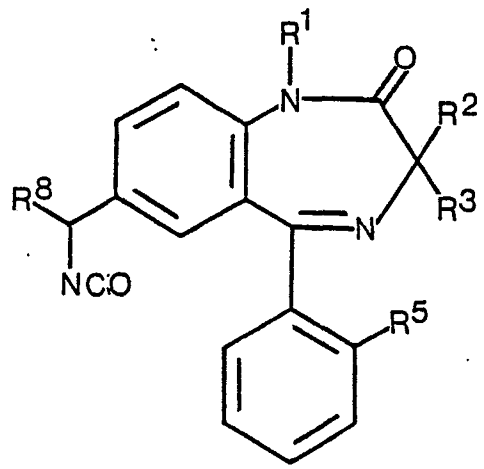

V

worin $R^1$ niederes Alkyl, $R^2$ und $R^3$ je Wasserstoff oder niederes Alkyl, $R^5$ Wasserstoff oder Halogen und $R^8$ Wasserstoff oder niederes Alkyl bedeuten.

12. Verbindungen gemäss einem der Ansprüche 1 bis 10 als pharmazeutische Wirkstoffe.

13. Verbindungen gemäss einem der Ansprüche 1 bis 10 als aldosteronantagonistische Wirkstoffe.

14. Verfahren zur Herstellung von Benzodiazepin-Derivaten gemäss einem der Ansprüche 1 bis 10, dadurch gekennzeichnet, dass man

a) ein Nitril der allgemeinen Formel

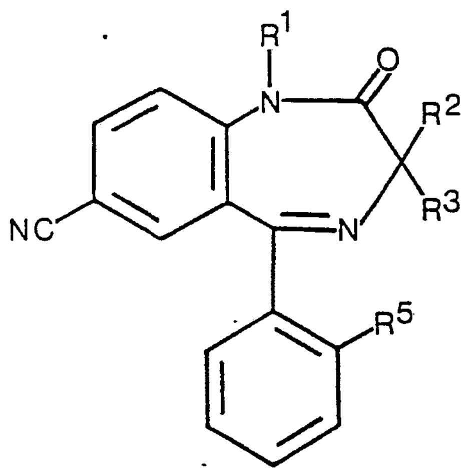

II

worin $R^1$, $R^2$, $R^3$ und $R^5$ die in Anspruch 1 angegebene Bedeutung besitzen,

zum entsprechenden primären Amin reduziert, oder

b) eine Carbonylverbindung der allgemeinen Formel

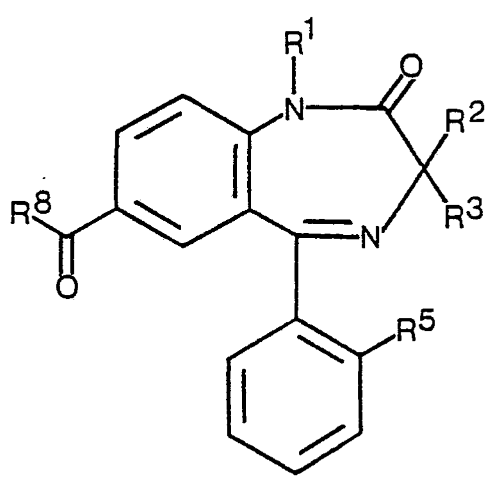

III

worin $R^1$, $R^2$, $R^3$, $R^5$ und $R^8$ die in Anspruch 1 angegebene Bedeutung besitzen,

mit Hydroxylamin behandelt, oder

c) eine Carbonylverbindung der obigen allgemeinen Formel III mit einem Amin der allgemeinen Formel

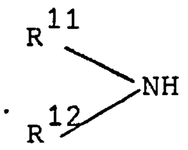 IV

worin $R^{11}$ und $R^{12}$ die in Anspruch 1 angegebene Bedeutung besitzen,

und einem Reduktionsmittel umsetzt, oder

d) in einer Verbindung der allgemeinen Formel

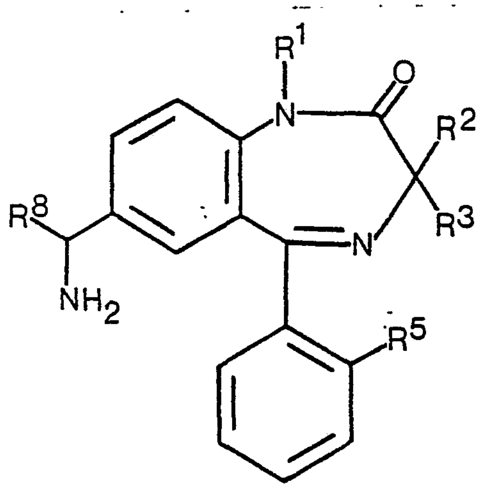 Ib'

worin $R^1$, $R^2$, $R^3$, $R^5$ und $R^8$ die in Anspruch 1 angegebene Bedeutung besitzen,

die primäre Aminogruppe mono- oder dialkyliert, oder

e) ein Oxim der allgemeinen Formel

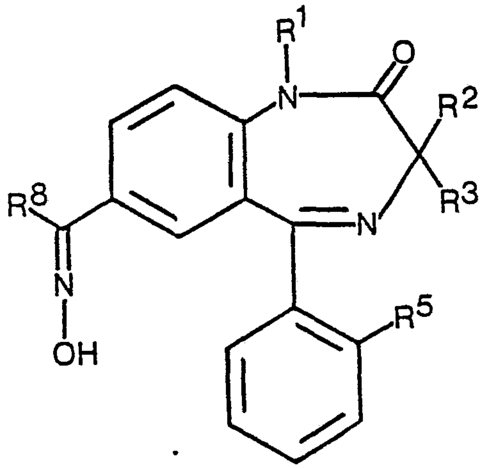

Ia

worin $R^1$, $R^2$, $R^3$, $R^5$ und $R^8$ die in Anspruch 1 angegebene Bedeutung besitzen,

zum entsprechenden primären Amin reduziert, oder

f) ein Isocyanat der allgemeinen Formel

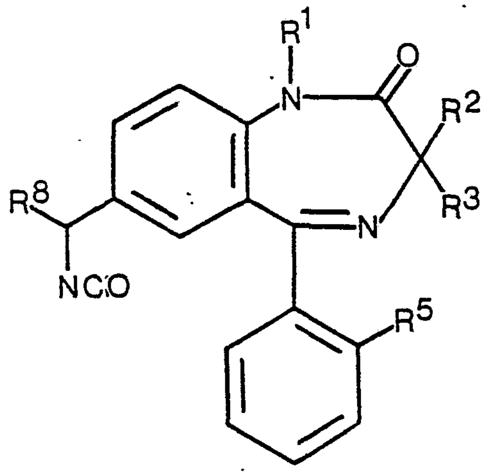

V

worin $R^1$, $R^2$, $R^3$, $R^5$ und $R^8$ die in Anspruch 1 angegebene Bedeutung besitzen,

mit einem Amin der allgemeinen Formel

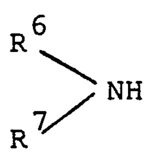

VI

worin $R^6$ und $R^7$ die in Anspruch 1 angegebene Bedeutung besitzen,

umsetzt, oder

g) ein Amin der allgemeinen Formel

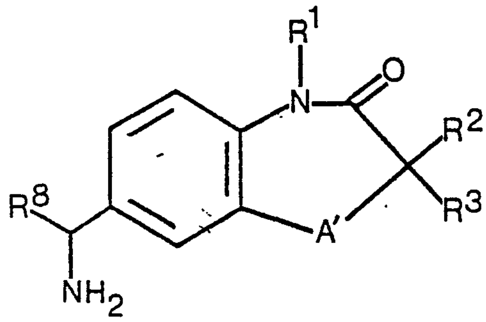

Ib

worin A' die in Anspruch 1 angegebene Gruppe (a) oder (b) bedeutet, und $R^1$, $R^2$, $R^3$ und $R^8$ die in Anspruch 1 angegebene Bedeutung besitzen,

mit einem Halogenid der allgemeinen Formel

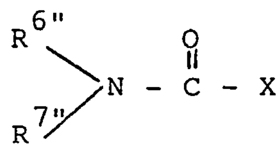

VII

worin X Halogen bedeutet, und entweder $R^{6''}$ und $R^{7''}$ je niederes Alkyl bedeuten oder $R^{6''}$ und $R^{7''}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13'}$ als Ringglied enthalten kann, wobei $R^{13'}$ niederes Alkyl bedeutet,

umsetzt, oder

h) ein Amin der obigen allgemeinen Formel Ib mit einem Isocyanat der allgemeinen Formel

$$R^{7'''} - NCO \qquad VIII$$

worin $R^{7'''}$ niederes Alkyl bedeutet,

umsetzt, oder

i) einen Alkohol der allgemeinen Formel

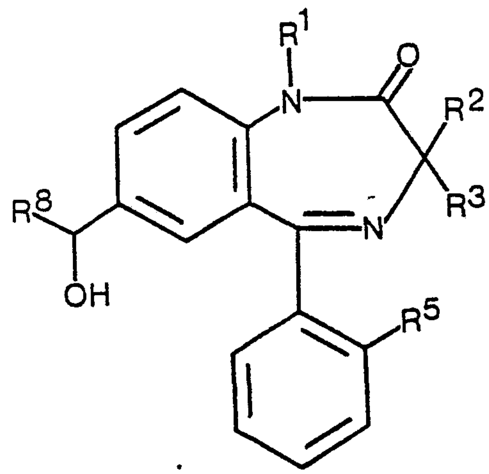

IX

worin $R^1$, $R^2$, $R^3$, $R^5$ und $R^8$ die in Anspruch 1 angegebene Bedeutung besitzen,

mit einer Verbindung der allgemeinen Formel

$$R^9 - L \qquad X$$

worin $R^9$ die in Anspruch 1 angegebene Bedeutung bebesitzt, und L eine Abgangsgruppe bedeutet,

veräthert, oder

j) einen Alkohol der obigen Formel IX mit einem einen Rest der allgemeinen Formel

$$R^{10} - \overset{O}{\overset{\|}{C}} - \qquad XI$$

worin $R^{10}$ die in Anspruch 1 angegebene Bedeutung besitzt,

liefernden Mittel umsetzt, oder

k) einen Alkohol der obigen allgemeinen Formel IX mit einem Isocyanat der allgemeinen Formel

$$R^{14} - NCO \qquad XII$$

worin $R^{14}$ die in Anspruch 1 angegebene Bedeutung besitzt,

umsetzt, oder

l) eine Iminoverbindung der allgemeinen Formel

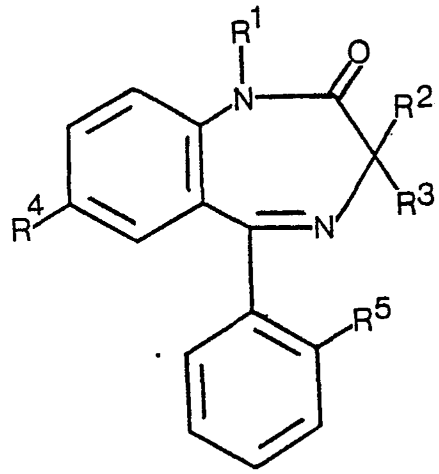

Ic

worin $R^1$, $R^2$, $R^3$, $R^4$ und $R^5$ die in Anspruch 1 angegebene Bedeutung besitzen,
zum entsprechenden Amin reduziert, oder

m) eine Verbindung der allgemeinen Formel I in ein pharmazeutisch annehmbares Säureadditionssalz überführt.

15. Arzneimittel, enthaltend eine Verbindung gemäss einem der Ansprüche 1 bis 10.

16. Aldosteronantagonisierende Arzneimittel, enthaltend eine Verbindung gemäss einem der Ansprüche 1 bis 10.

Patentansprüche
"für OESTERREICH"

1. Verfahren zur Herstellung von Benzodiazepin-Derivaten der allgemeinen Formel

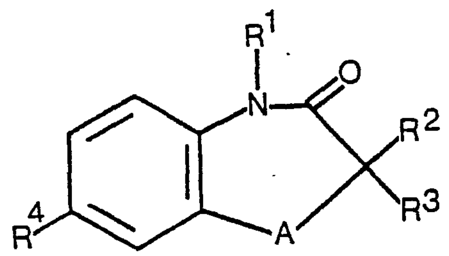

I

worin A die Gruppe

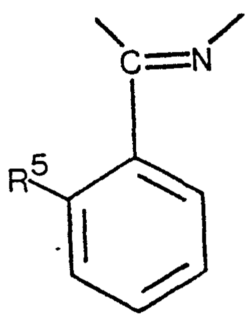

(a),

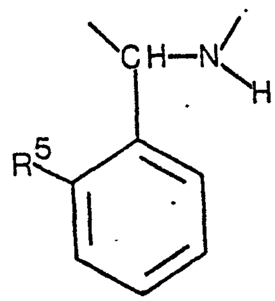

(b) oder

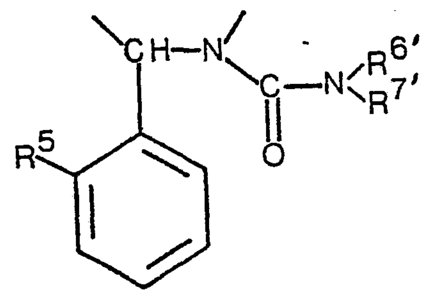

(c),

$R^1$ niederes Alkyl, $R^2$ und $R^3$ je Wasserstoff oder niederes Alkyl, $R^4$ die Gruppe

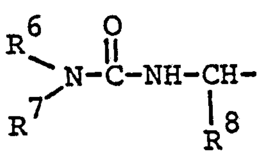

,

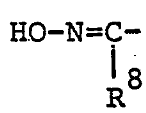

,

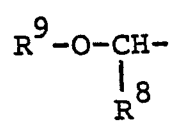

,

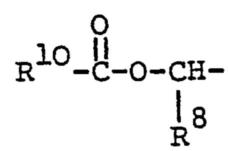

,

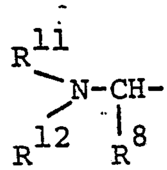

oder

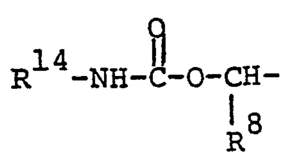

,

$R^5$ Wasserstoff oder Halogen, $R^8$ Wasserstoff oder niederes Alkyl, $R^9$ niederes Alkyl oder niederes Alkoxyalkyl, $R^{10}$ niederes Alkyl, $R^{11}$ Wasserstoff, niederes

Alkyl oder niederes Hydroxyalkyl, $R^{12}$ Wasserstoff oder niederes Alkyl und $R^{14}$ niederes Alkyl oder Aryl bedeuten, und entweder $R^6$ Wasserstoff oder niederes Alkyl und $R^7$ niederes Alkyl oder niederes Hydroxyalkyl bedeuten oder $R^6$ und $R^7$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13}$ als Ringglied enthalten kann, wobei $R^{13}$ Wasserstoff oder niederes Alkyl bedeutet, und entweder $R^{6'}$ Wasserstoff oder niederes Alkyl und $R^{7'}$ niederes Alkyl bedeuten oder $R^{6'}$ und $R^{7'}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13'}$ als Ringglied enthalten kann, wobei $R^{13'}$ niederes Alkyl bedeutet, mit der Massgabe, dass $R^4$ die Gruppe $R^{6'}R^{7'}N-CO-NH-CH(R^8)-$ bedeutet, wenn A die Gruppe (c) bedeutet,

und von pharmazeutisch annehmbaren Säureadditionssalzen davon, dadurch gekennzeichnet, dass man

a) ein Nitril der allgemeinen Formel

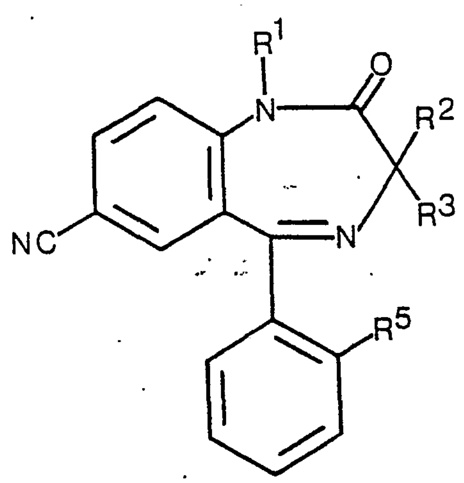

II

worin $R^1$, $R^2$, $R^3$ und $R^5$ obige Bedeutung besitzen, zum entsprechenden primären Amin reduziert, oder

b) eine Carbonylverbindung der allgemeinen Formel

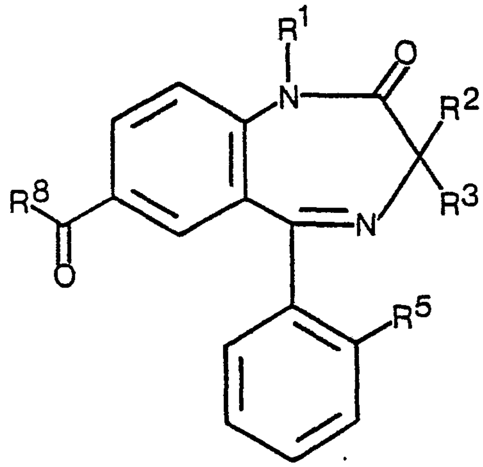

III

worin $R^1$, $R^2$, $R^3$, $R^5$ und $R^8$ obige Bedeutung besitzen, mit Hydroxylamin behandelt, oder

c) eine Carbonylverbindung der obigen allgemeinen Formel III mit einem Amin der allgemeinen Formel

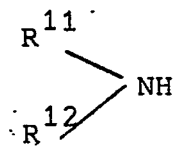

IV

worin $R^{11}$ und $R^{12}$ obige Bedeutung besitzen, und einem Reduktionsmittel umsetzt, oder

d) in einer Verbindung der allgemeinen Formel

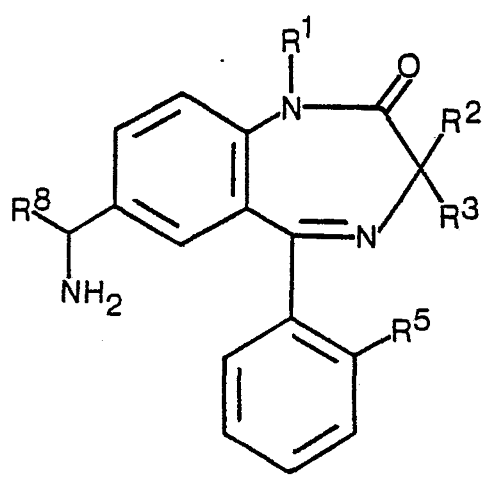

Ib'

worin $R^1$, $R^2$, $R^3$, $R^5$ und $R^8$ obige Bedeutung besitzen,

die primäre Aminogruppe mono- oder dialkyliert, oder

e) ein Oxim der allgemeinen Formel

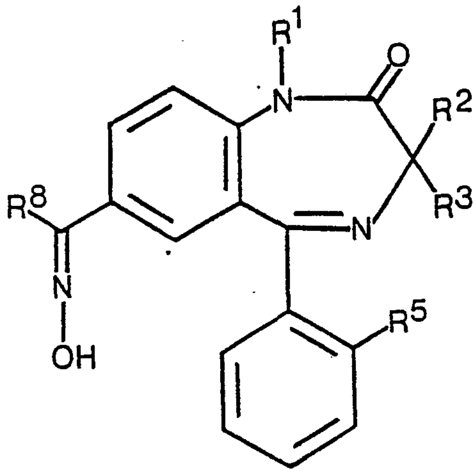

Ia

worin $R^1$, $R^2$, $R^3$, $R^5$ und $R^8$ obige Bedeutung besitzen, zum entsprechenden primären Amin reduziert, oder

f) ein Isocyanat der allgemeinen Formel

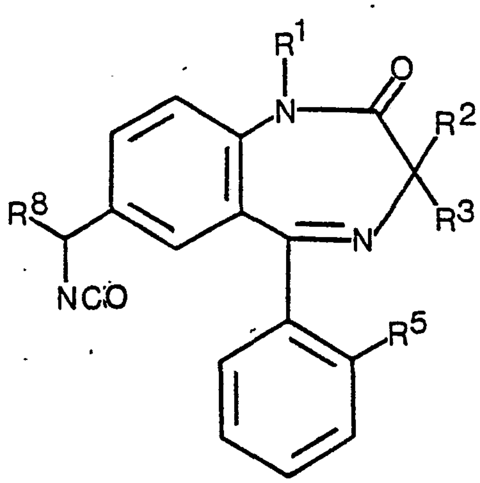

V

worin $R^1$, $R^2$, $R^3$, $R^5$ und $R^8$ obige Bedeutung besitzen, mit einem Amin der allgemeinen Formel

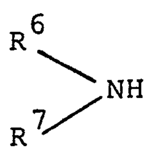

VI

worin $R^6$ und $R^7$ obige Bedeutung besitzen,

umsetzt, oder

g) ein Amin der allgemeinen Formel

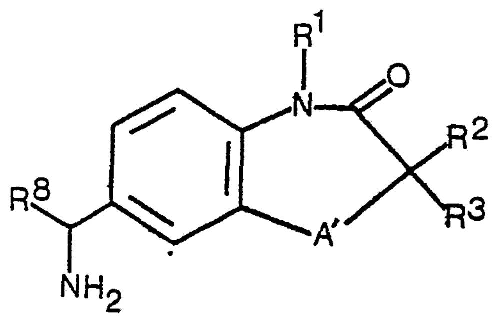

Ib

worin A' die Gruppe (a) oder (b) bedeutet, und $R^1$, $R^2$, $R^3$ und $R^8$ obige Bedeutung besitzen,

mit einem Halogenid der allgemeinen Formel

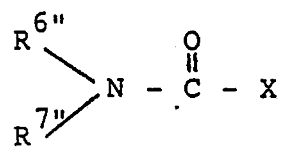

VII

worin X Halogen bedeutet, und entweder $R^{6''}$ und $R^{7''}$ je niederes Alkyl bedeuten oder $R^{6''}$ und $R^{7''}$ zusammen mit dem Stickstoffatom einen 3- bis 7-gliedrigen Heterocyclus bedeuten, der - falls er mindestens 5-gliedrig ist - ein Sauerstoff- oder Schwefelatom oder einen Rest der Formel $>N-R^{13'}$ als Ringglied enthalten kann, wobei $R^{13'}$ niederes Alkyl bedeutet,

umsetzt, oder

h) ein Amin der obigen allgemeinen Formel Ib mit einem Isocyanat der allgemeinen Formel

$$R^{7'''} - NCO \qquad VIII$$

worin $R^{7'''}$ niederes Alkyl bedeutet,

umsetzt, oder

i) einen Alkohol der allgemeinen Formel

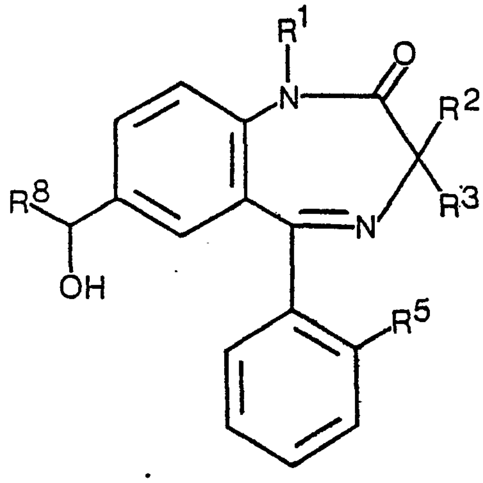

IX

worin $R^1$, $R^2$, $R^3$, $R^5$ und $R^8$ obige Bedeutung besitzen, mit einer Verbindung der allgemeinen Formel

$$R^9 - L \qquad X$$

worin $R^9$ obige Bedeutung besitzt, und L eine Abgangsgruppe bedeutet,

veräthert, oder

j) einen Alkohol der obigen Formel IX mit einem einen Rest der allgemeinen Formel

$$R^{10} - \overset{O}{\overset{\|}{C}} - \qquad XI$$

worin $R^{10}$ obige Bedeutung besitzt,

liefernden Mittel umsetzt, oder

k) einen Alkohol der obigen allgemeinen Formel IX mit einem Isocyanat der allgemeinen Formel

$$R^{14} - NCO \qquad XII$$

worin $R^{14}$ obige Bedeutung besitzt,

umsetzt, oder

l) eine Iminoverbindung der allgemeinen Formel

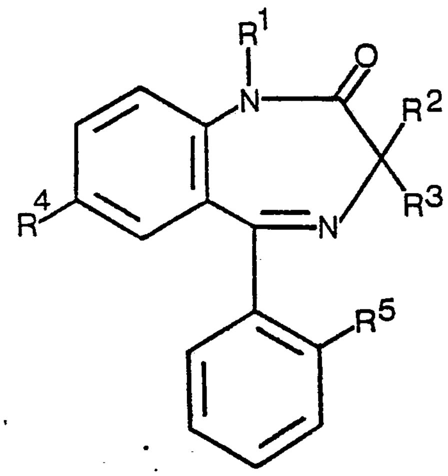

Ic

worin $R^1$, $R^2$, $R^3$, $R^4$ und $R^5$ obige Bedeutung besitzen,

zum entsprechenden Amin reduziert, oder

m) eine Verbindung der allgemeinen Formel I in ein pharmazeutisch annehmbares Säureadditionssalz überführt.

2. Verfahren gemäss Anspruch 1, dadurch gekennzeichnet, dass man Verbindungen der in Anspruch 1 definierten allgemeinen Formel I herstellt, worin entweder $R^1$ und $R^2$ beide Methyl oder $R^1$ Aethyl und $R^2$ Wasserstoff bedeuten, wenn $R^3$ Wasserstoff, $R^4$ die Gruppe $HO-N=C(CH_3)-$, A die Gruppe (a) und $R^5$ Fluor bedeuten.

3. Verfahren gemäss Anspruch 1, dadurch gekennzeichnet, dass man Verbindungen der in Anspruch 1 definierten allgemeinen Formel I herstellt, worin $R^2$ und $R^3$ beide niederes Alkyl bedeuten, wenn $R^4$ die Gruppe $HO-N=C(R^8)-$ und A die Gruppe (c) bedeuten.

4. Verfahren gemäss einem der Ansprüche 1 bis 3, dadurch gekennzeichnet, dass man Verbindungen der in Anspruch 1 definierten allgemeinen Formel I herstellt, worin A die Gruppe (a) oder (b), $R^1$ Methyl oder Aethyl, $R^2$ und $R^3$ Wasserstoff oder Methyl, $R^4$ die Gruppe $R^6R^7N-CO-NH-CH(R^8)-$, $HO-N=C(R^8)-$, $R^9-O-CH(R^8)-$ oder $R^{11}R^{12}N-CH(R^8)-$, $R^5$ Wasserstoff oder Fluor und $R^8$ Wasserstoff oder Methyl bedeuten.

5. Verfahren gemäss Anspruch 4, dadurch gekennzeichnet, dass man 1-Aethyl-5-(o-fluorphenyl)-1,3-dihydro-7-[1-(methoxymethoxy)äthyl]-2H-1,4-benzodiazepin-2-on herstellt.

6. Verfahren gemäss Anspruch 4, dadurch gekennzeichnet, dass man 1-Aethyl-5-(o-fluorphenyl)-1,3,4,5-tetrahydro-7-[1-(hydroxyimino)äthyl]-2H-1,4-benzodiazepin-2-on herstellt.

7. Verfahren gemäss Anspruch 4, dadurch gekennzeichnet, dass man 1-Aethyl-5-(o-fluorphenyl)-1,3,4,5-tetrahydro-7-/1-[(2-hydroxyäthyl)amino]äthyl/-2H-1,4-benzodiazepin-2-on herstellt.

8. Verfahren gemäss Anspruch 4, dadurch gekennzeichnet, dass man 7-[1-(Dimethylamino)äthyl]-1-äthyl-5-(o-fluorphenyl)-1,3,4,5-tetrahydro-2H-1,4-benzodiazepin-2-on herstellt.

9. Verfahren gemäss Anspruch 4, dadurch gekennzeichnet, dass man 7-(1-Aminoäthyl)-5-(o-fluorphenyl)-1,3-dihydro-1,3-dimethyl-2H-1,4-benzodiazepin-2-on herstellt.

10. Verfahren gemäss Anspruch 4, dadurch gekennzeichnet, dass man 1-[(2,3-Dihydro-1-methyl-2-oxo-5-phenyl-1H-1,4-benzodiazepin-7-yl)methyl]-3-(2-hydroxyäthyl)harnstoff herstellt.